# EUROPÄISCHE PATENTSCHRIFT

(11) **EP 0 345 653 B1**
(45) Veröffentlichungstag und Bekanntmachung des Hinweises auf die Patenterteilung: **26.10.1994**
(21) Anmeldenummer: 89109979.8
(22) Anmeldetag: 02.06.1989
(51) Int. Cl.: C07F 15/00, C07F 15/06, B01J 31/16

(54) **Übergangsmetallkomplexe**
Transition metal complex
Complexe de métal de transition

(30) Priorität: 08.06.1988 DE 3819487
(43) Veröffentlichungstag der Anmeldung: 13.12.1989
(73) Patentinhaber: BASF Aktiengesellschaft, 67063 Ludwigshafen (DE)
(72) Erfinder: Bertleff, Werner, Dr., D-6806 Viernheim (DE); Koeffer, Dieter, Dr., D-6940 Weinheim (DE); Klaeui, Wolfgang, Prof. Dr., NL-6291 CL Vaals (NL); Song, Choong-Eui, (Lee, Chung-Ho),Dr., Sang-A-A-pt. 2 Dong 501 Seoul (KR)

(56) Entgegenhaltungen:
- EP-A- 0 138 141
- BE-A- 651 407
- ANGEWANDTE CHEMIE, Band 97, Nr. 8, 1985, Seiten 697-698, Weinheim, DE; W. KLÄUIet al.: "Dreizähnige Sauerstoff-Liganden als Cyclopentadienyl-Äquivalente:Struktur und Eigenschaften von [LRh(mu-CO)3RhL], L-=[(C5H5),Co[P(0)R2]3]-"

## Beschreibung

Die Vorliegende Erfindung betrifft neue Übergangsmetallkomplexe der allgemeinen Formel I

[A]^{x-} [Q]⁺ₓ I

in der Q für das Äquivalent eines Kations steht, x einen wert von 0 bis 2 hat und A einem Übergangsmetallkomplex der allgemeinen Formel 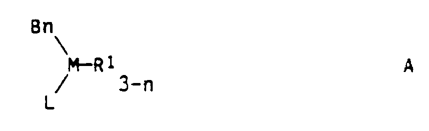
entspricht, in welcher die Variablen folgende Bedeutung haben:
- n: 1 bis 3
- M: positiv geladenes Cobalt, Rhodium, Iridium oder Ruthenium
- B: gleiche oder verschiedene Gruppen der allgemeinen Formel II 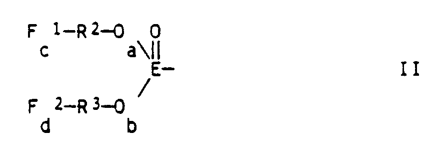 in der die Variablen wie folgt definiert sind:
- E: Phosphor oder Arsen,
- O: Sauerstoff,
- R², R³: C₁- bis C₂₀-Alkylengruppen, die ihrerseits bis zu Zwei Cycloalkyl-, Heterocycloalkyl-, Aryl- oder Heteroarylgruppen mit insgesamt bis Zu 15 Ringatomen tragen können und wobei die Reste R² und R³ durch Sauerstoff, Schwefel oder (-NR⁴)-Gruppen unterbrochen sein können, wobei R⁴ Wasserstoff oder C₁- bis C₄-Alkyl bedeutet und wobei die Heteroatome bzw. Heteroatomgruppierungen jeweils durch mindestens 2 Kohlenstoffatome getrennt sind und wobei die von zwei verschiedenen (-NR⁴)Gruppierungen stammenden Reste R⁴ auch miteinander Zu einem 5- oder 6-gliedrigen Ring verbunden sein können,
- F¹, F²: Alkenyl, Alkinyl, C₅- bis C₁₂-Cycloalkadienyl-, Nitril-, Amino-, C₁- bis C₄-Mono- und Dialkylamino, C₁- bis C₄-Acyl-, Hydroxyl-, C₁- bis C₄-Alkoxy-, Aryloxy-, C₁- bis C₄-Alkylsulfido-, Arylsulfido, C₁- bis C₄-Dialkyl- und Diarylphosphido und/oder Carboxylato sind, und falls c oder d gleich 0 sind, für Wasserstoff stehen.
- a - d: 0 oder 1, mit der Maßgabe, daß a+b>0 und c+d>0 sind
- L: 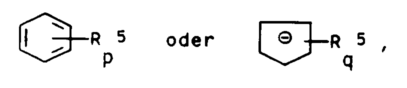 wobei R⁵ für gleiche oder verschiedene Reste aus der Gruppe C₁- bis C₄-Alkyl oder Phenyl steht, p eine der ganzen Zahlen 0 bis 6 und q eine der ganzen Zahlen 0 bis 5 bedeutet,
- R¹: Fluor, Chlor, Brom, Iod, Cyanid, Isocyanid, Cyanat, Isocyanat, Thiocyanat, Isothiocyanat C₁- bis C₄-Alkyl, C₁- bis C₄-Trialkyl- oder Triarylphosphit.

Weiterhin betrifft die vorliegende Erfindung Verfahren zur katalytischen Hydrierung ungesättigter Verbindungen, zur Hydroformylierung von Alkenen und zur Cyclotrimerisierung von Acetylenverbindungen, wobei diese Übergangsmetallkomplexe als Katalysatoren eingesetzt oder in situ erzeugt werden.

Übergangsmetallhomogenkatalysatoren haben in den vergangenen Jahren eine große Bedeutung in den Verfahren der industriellen organischen Chemie, insbesondere bei Hydrierungs- und Hydroformylierungsverfahren erlangt. Jedoch bereitet bei der Anwendung dieser Homogenkatalysatoren das Problem der Produktabtrennung unter Erhalt der Katalysatoraktivität immer noch große Schwierigkeiten. Insbesondere Homogenkatalysatoren, die ein Übergangsmetall als aktive Komponente enthalten, sind häufig unter den Bedingungen der Aufarbeitung des Reaktionsgemisches nicht stabil, so daß dabei der Katalysator teilweise inaktiviert wird. Weiterhin ist die vollständige Abtrennung der oftmals teuren, edelmetallhaltigen Homogenkatalysatoren vom Produkt schwierig zu bewerkstelligen. Die Aufarbeitung des Reaktionsgemisches führt folglich zu Verlusten durch Austrag des Katalysators mit dem Produkt.

In der Vergangenheit wurde deshalb versucht, den Katalysator durch geeignete Liganden, z.B. sulfonierte Phosphine, in einer flüssigen Phase zu fixieren, die mit der produktführenden flüssigen Phase nicht mischbar ist. Hierbei erfolgt die Produktabtrennung wie in DE-B 26 27 354, EP-B 103 810 und DE-A 35 34 314 beschrieben, durch Phasentrennung. Obwohl dadurch eine thermische Belastung des Katalysators bei der Aufarbeitung weitgehend vermieden wird, findet bei der Aufarbeitung, infolge der Abspaltung der Sulfonsäuregruppen oder der Spaltung der Phosphor-Arylbindung, dennoch eine Desaktivierung des Katalysators statt.

EP-A 138 141 betrifft ein Verfahren zur selektiven Hydrierung von C-C-Doppelbindungen in Gegenwart von reduzierbaren, stickstoffhaltigen Gruppe mit Hilfe von katalytisch wirksamen Rutheniumkomplesen, der Formel

RuXL₁ (L₂)₂

in der
- X: für Chlor, Brom, Jod oder Wasserstoff steht,
- L₁: für einen aromatischen Liganden der Formel 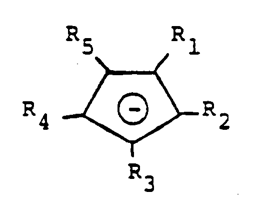 steht, in der R₁ bis R₅ gleich oder verschieden sein können und für Wasserstoff, Methyl, Ethyl oder Phenyl stehen, wobei auch je zwei benachbarte Reste aus der Gruppe R₁ bis R₅ gemeinsam einen Kohlenwasserstoffrest derart bilden können, daß L₁ insgesamt ein kondensiertes Ringsystem darstellt, das gegebenenfalls unter Reaktionsbedingungen inerte Substituenten enthält und
- L₂: für einen Liganden aus der Gruppe der tertiären Organophosphor-, Organoarsen- und Organoantimon-Verbindungen steht oder
- (L₂)₂: für einen zweizähnigen Bisphosphan-Liganden steht.

Der vorliegenden Erfindung lag deshalb die Aufgabe zugrunde, Übergangsmetallkomplexe zur Verfügung zu stellen, die als Homogenkatalysatoren zur Hydrierung ungesättigter Verbindung, zur Hydroformylierung von Alkenen und zur Cyclotrimerisierung von Acetylenverbindungen bzw. zur Herstellung dieser Katalysatoren geeignet sind. Diese Katalysatoren sollten sich durch eine gute katalytische Aktivität sowie durch eine hohe Temperaturstabilität auszeichnen und unter den Bedingungen der Aufarbeitung des Reaktionsgemisches chemisch stabil sein. Diese Übergangsmetallverbindungen sollten sich außerdem in ihrer chemischen Struktur leicht variieren lassen, um ihre Anwendung je nach Bedarf in Wasser, polaren oder unpolaren organischen Lösungsmitteln zu ermöglichen.

Dementsprechend wurden neue Übergangsmetallkomplexe der allgemeinen Formel I gefunden,

[A]^{x-} [Q]⁺ₓ I

in der Q für das Äquivalent eines Kations steht, x einen Wert von 0 bis 2 hat und A einem Übergangsmetallkomplex der allgemeinen Formel 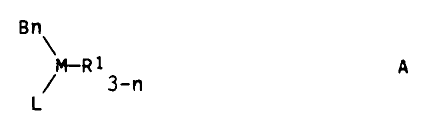
entspricht, in welcher die Variablen folgende Bedeutung haben:
- n: 1 bis 3
- M: positiv geladenes Cobalt, Rhodium, Iridium oder Ruthenium
- B: gleiche oder verschiedene Gruppen der allgemeinen Formel II 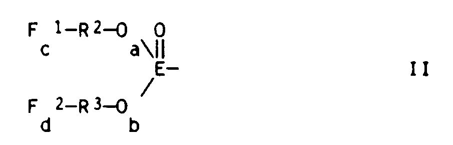 in der die Variablen wie folgt definiert sind:
- E: Phosphor oder Arsen,
- O: Sauerstoff,
- R², R³: C₁- bis C₂₀-Alkylengruppen, die ihrerseits bis zu zwei Cycloalkyl-, Heterocycloalkyl-, Aryl- oder Heteroarylgruppen mit insgesamt bis zu 15 Ringatomen tragen können und wobei die Reste R² und R³ durch Sauerstoff, Schwefel oder (-NR⁴)-Gruppen unterbrochen sein können, wobei R⁴ Wasserstoff oder C₁- bis C₄-Alkyl bedeutet und wobei die Heteroatome bzw. Heteroatomgruppierungen jeweils durch mindestens 2 Kohlenstoffatome getrennt sind und wobei die von zwei verschiedenen (-NR⁴)Gruppierungen stammenden Reste R⁴ auch miteinander zu einem 5- oder 6-gliedrigen Ring verbunden sein können,
- F¹, F²: Alkenyl, Alkinyl, C₅- bis C₁₂-Cycloalkadienyl, Nitril-, Amino-, C₁- bis C₄-Mono- und Dialkylamino, C₁- bis C₄-Acyl-, Hydroxyl-, C₁- bis C₄-Alkoxy-, Aryloxy-, C₁- bis C₄-Alkylsulfido-, Arylsulfido, C₁- bis C₄-Dialkyl- und Diarylphosphido und/oder Carboxylato sind und falls c oder d gleich 0 sind, für Wasserstoff stehen,
- a - d: 0 oder 1, mit der Maßgabe, daß a+b>0 und c+d>0 sind
- L: 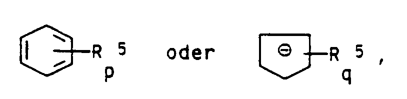 wobei R⁵ für gleiche oder verschiedene Reste aus der Gruppe C₁- bis C₄-Alkyl oder Phenyl steht, p eine der ganzen Zahlen 0 bis 6 und q eine der ganzen Zahlen 0 bis 5 bedeutet,
- R¹: Fluor, Chlor, Brom, Iod, Cyanid, Isocyanid, Cyanat, Isocyanat, Thiocyanat, Isothiocyanat, C₁- bis C₄-Alkyl, C₁- bis C₄-Trialkyl- oder Triarylphosphit.

Insbesondere betrifft die vorliegende Erfindung Übergangsmetallkomplexe der allgemeinen Formel III

[L-Co-B₃]⁻[Q]⁺ III

wobei E der Gruppierung B Phosphor bedeutet,
Übergangsmetallkomplexe der allgemeinen Formel III, in denen E der Gruppierung II Phosphor bedeutet, C gleich 0 ist und R³ ausgewählt ist aus C₁- bis C₁₆-Alkylen,
Übergangsmetallkomplexe der allgemeinen Formel IV, 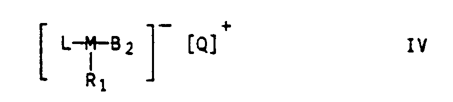
in denen M ausgewählt ist aus Rhodium, Iridium und Rutheniumkationen und wobei E der Gruppierung II Phosphor bedeutet sowie Übergangsmetallkomplexe der allgemeinen Formel IV, in denen M ausgewählt ist aus Rhodium, Iridium und Rutheniumkationen, E der Gruppierung II Phosphor bedeutet, C gleich 0 ist und R³ ausgewählt ist aus C₁- bis C₁₆-Alkylen.

Die vorliegende Erfindung betrifft auch Übergangsmetallkomplexe der allgemeinen Formel I, in denen [Q]⁺ ein Komplexkation des Typs V

[M^{Q}Z]^{x⊕} V

bedeutet, worin
X einen Wert von 0, 1 oder 2 hat,
M^{Q} für Rhodium, Iridium, Ruthenium, Chrom, Wolfram, Molybdän, Mangan, Rhenium oder Kupfer steht,
Z für einen der mehrzähnigen Liganden C₇-Cycloalkatrien, C₅- bis C₁₂-Cycloalkadien, C₄- bis C₈-Alkadien, Aren oder einen C₂- bis C₆-Alkylendiphosphido-Liganden der allgemeinen Formel VI

R⁶-P-(CH₂)₁₋₆-P-R⁶ VI,

worin R⁶ einen C₁- bis C₄-Alkyl- und/oder Phenylrest bedeutet,
und/oder für 1 bis 3 gleiche oder verschiedene der einzähnigen Liganden Carbonyl, Nitrosyl, Chlor, Brom, Iod, Nitril, Isonitril, C₁- bis C₄-Alkyl, C₁- bis C₄-Acyl, C₁- bis C₄-Alken, C₁- bis C₄-Alkin, C₁- bis C₄-Trialkylphosphino, Triarylphosphino- und/oder Carbin-Liganden steht.

Weiterhin betrifft die vorliegende Erfindung Übergangsmetallkomplexe der allgemeinen Formel I, in denen [Q]⁺ für ein Komplexdikation der allgemeinen Formel VII

[M^{Q}-K-M^{Q}]²⁺ VII

steht, worin K für 1 bis 3 der Brückenliganden Carbonyl, C₄- bis C₆-Alkadien, C₈- bis C₁₂-Cycloalkadien, C₁- bis C₄-Alkylisonitril, C₁- bis C₄-Dialkylphosphido, Diarylphosphido und/oder C₁- bis C₆-Alkylendiphosphido-Liganden der allgemeinen Formel VI stehen.

Außerdem betrifft die vorliegende Erfindung Verfahren zur katalytischen Hydrierung ungesättigter Verbindungen, zur Hydroformylierung von Alkenen und zur Cyclotrimerisierung von Acetylenverbindungen, wobei diese Übergangsmetallkomplexe als Katalysatoren eingesetzt oder in situ erzeugt werden.

Die neuen Übergangsmetallkomplexe der allgemeinen Formel I

[A]^{x-} [Q]⁺ₓ I

sind aufgebaut aus einer Komplexverbindung A der allgemeinen Formel 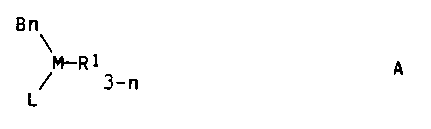
die neutral (X=0) oder einfach oder zweifach negativ geladen sein kann und gegebenenfalls (X>0) einem Gegenion Q, wobei Q für das Äquivalent eines Kations steht. Das zentrale Metallion dieser Komplexe, das entweder ein Cobalt-, Rhodium-, Iridium- oder Rutheniumion der Oxidationsstufe II oder III sein kann, ist im allgemeinen mit vier Liganden koordiniert, nämlich L, R¹ und B, wobei die Liganden L und R¹ lediglich der koordinativen und elektronischen Absättigung des Zentralions dienen, während der oder die Liganden B zusätzlich als komplexierendes Agens für die Kation-Äquivalente Q fungieren können.

L ist ein organischer Ligand, der in der Regel mehrzähnig ist, beispielsweise Cyclopentadienyl, Monomethylcyclopentadienyl, Pentamethylcyclopentadienyl, n-Butylcyclopentadienyl, Penta-n-butylcyclopentadienyl, Phenylcyclopentadienyl, Pentaphenylcyclopentadienyl, 1,3,4-Triphenylcyclopentadienyl, 1,3,4-Triphenyl-2,5-dimethyl-cyclopentadienyl, Benzol, Toluol, Mesitylen, Hexamethylbenzol, Hexa-n-butylbenzol, Biphenyl oder 1,3,5-Triphenylbenzol. Dabei kann der Substitutionsgrad sowie das Substitutionsmuster der Cyclopentadienylliganden oder Alkylbenzolliganden beliebig gewählt werden. Der Substitutionsgrad und das Substitutionsmuster der Phenyl- bzw. Phenyl-alkyl-benzolliganden ist insoweit beliebig als er nicht durch ungünstige sterische Wechselwirkungen der Substituenten untereinander ausgeschlossen ist. Bevorzugte Liganden L sind aufgrund ihrer leichten Zugänglichkeit Cyclopentadienyl, Methylcyclopentadienyl, Pentamethylcyclopentadienyl, Benzol und Hexamethylbenzol. Für die Übergangsmetallkomplexe in denen A für die Komplexanionen in der allgemeinen Formel III steht, werden insbesondere die Cyclopentadienyl-, Methylcyclopentadienyl- und Pentamethylcyclopentadienylliganden bevorzugt. Bei den Übergangsmetallkomplexen in denen A den Komplexanionen in der allgemeinen Formel IV entspricht können sich außerdem Benzol- und Hexamethylbenzolliganden als besonders vorteilhaft erweisen. Es können jedoch auch andere Liganden als die hier genannten verwendet werden, sofern sie in der Lage sind, die Valenzschale des Zentralions auf 16 oder 18 Elektronen aufzufüllen.

Die Zahl der Liganden R¹ mit denen das Zentralion M koordiniert ist hängt von der Anzahl der Liganden B ab. Ist das Zentralion mit 3 Liganden B koordiniert, so ist dessen koordinative und elektronische Absättigung durch einen Liganden R¹ nicht mehr erforderlich. Sind hingegen nur 1 oder 2 Liganden B mit dem Zentralion M koordiniert, so ist die Anwesenheit von 2 bzw. 1 Liganden R¹ im Komplex A für dessen Stabilität notwendig. Da der oder die Liganden R¹ allein der koordinativen und elektronischen Absättigung des Zentralatoms M dienen, ist ihre Auswahl beliebig. Beispielsweise seien für die Liganden R¹ die Gruppen Trialkylphosphit, Triarylphosphit, C₁- bis C₄-Alkyl, die Halogenide Fluor, Chlor, Brom und Iod sowie die Pseudohalogenide Nitril, Isonitril, Cyanat, Isocyanat, Thiocyanat und Isothiocyanat genannt. Es können jedoch auch andere Liganden verwendet werden. Sind im Komplex A zwei Liganden R¹ mit dem Zentralion koordiniert, so können diese selbstverständlich gleich oder verschieden sein. Es ist auch möglich, daß anstelle zweier einzähniger Liganden R¹ ein zweizähniger Ligand R¹ verwendet wird.

Im Gegensatz zu den Liganden L und R¹ sind der oder die Liganden B für die katalytischen Eigenschaften der Übergangsmetallkomplexe I essentiell. In den Komplexen A, die den neutralen oder anionischen Teil der Übergangsmetallkomplexe I darstellen, ist das Zentralion M mit 1 bis 3, vorzugsweise 2 oder 3, gleichen oder verschiedenen Liganden B, welche für eine Gruppierung der allgemeinen Formel II stehen, 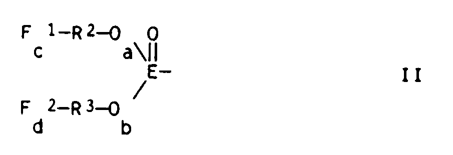
koordiniert. Da E ein Phosphor- oder Arsenatom sein kann, handelt es sich bei den Liganden B mithin um Phosphon- oder Arsonsäureester, falls a und b jeweils die Zahl 1 bedeuten oder um Phosphin- oder Arsinsäureester, falls a oder b für die Zahl 0 steht, d.h. die Phosphor- oder Arsenatome in B können direkt und/oder über ein Sauerstoffatom mit gleichen oder verschiedenen Resten R² und R³ verbunden sein.

R² und R³ sind C₁- bis C₂₀-Alkylengruppen oder falls c oder d die Zahl 0 bedeuten, Alkylgruppen. Bevorzugt sind Alkylengruppen mit 1 bis 16 Kohlenstoffatomen.

Die Alkylengruppen können ihrerseits bis zu zwei Cycloalkyl-, Heterocycloalkyl-, Aryl- oder Heteroarylgruppen als Substituenten tragen. Diese Ringsysteme können bis zu 15 Ringatome umfassen.

Als Cycloalkylsubstituenten können prinzipiell alle C₃- bis C₁₅-Cycloalkylsysteme verwendet werden. Die Verwendung höhergliedriger Cycloalkylsysteme ist ebenfalls möglich, führt jedoch hinsichtlich der katalytischen Aktivität und der chemischen Eigenschaften der damit hergestellten erfindungsgemäßen Verbindungen zu keinen weiteren Vorteilen. Die Cycloalkylsysteme können monocyclisch, annelliert oder verbrückt sein.

Beispielsweise genannt seien die Cyclopropyl-, Cyclohexyl-, Decalinyl-, Indanyl-, Cyclooctyl- und die Norbornylgruppe. Besonders bevorzugt werden die Cyclopropyl, die Cyclopentyl- und die Cyclohexylgruppe verwendet.

Die Arylsubstituenten können einfache aromatische Systeme wie Phenyl, Biphenyl oder Terphenyl, aber auch annellierte Systeme wie Naphthalinyl, Anthracenyl, Phenanthrenyl oder Indenyl sein, bevorzugt sind jedoch Phenyl und Naphthalinyl.

Als Heteroarylsubstituenten können sowohl einfache 5- bis 6-gliedrige aber auch annellierte aromatische Systeme dienen, welche eines der Atome Sauerstoff oder Schwefel und/oder 1 oder 2 Stickstoffatome oder 1, 2 oder 3 Stickstoffatome pro Ring enthalten. Beispielhaft seien hier einige vorteilhaft verwendbare Hetarylgruppen genannt: Furanyl-, Thiophenyl-, Pyrrollyl-, Oxazolyl-, Thiazolyl-, Imidazolyl-, Isoxazolyl-, Pyrazolyl-, Triazolyl-, Pyridyl-, Pyrazinyl-, Pyrimidinyl-, Pteridinyl-, Phenanthrolyl-, Bipyridinyl- oder Chinolinyl-.

Weiterhin können die Reste R² und/oder R³ Heterocycloalkylgruppen tragen. Als solche Gruppen können 3- bis 4-gliedrige Heterocyclen mit einem, 5-gliedrige Heterocyclen mit einem oder zweien, 6- bis 15-gliedrige monocyclische aber auch annellierte oder verbrückte tri- oder tetracyclische Systeme mit einem oder bevorzugt mehreren der Heteroatome Sauerstoff, Schwefel, Stickstoff und/oder Phosphor verwendet werden. Als derartige Gruppen seien beispielsweise Oxiranyl-, Aziridinyl-, Tetrahydrofuranyl, Pyrrolidinyl-, Dithianyl-, Dioxanyl-, Piperidinyl-, Piperazinyl- und Diazabicycloundecanyl genannt.

Die Reste R² und R³ können auch durch Sauerstoff, Schwefel oder (NR⁴)-Gruppen unterbrochen sein, wobei R⁴ Wasserstoff oder C₁- bis C₄-Alkyl bedeutet, und wobei diese Heteroatome bzw. Heteroatomgruppierungen jeweils durch mindestens 2 Kohlenstoffatome getrennt sind. Als derartige Heteroalkylenreste werden bevorzugt Heteroalkylenketten vewendet, die aus Ethylenglykol- oder gegebenenfalls alkylierten Ethylendiamin-Oligomeren aufgebaut sind. Natürlich können die Heteroalkylketten auch mehrere voneinander verschiedene Heteroatome in der Kette enthalten, aufgrund ihrer leichten Zugänglichkeit sind jedoch Heteroalkylketten, welche nur eine Heteroatomspezies enthalten, bevorzugt.

Enthält die Heteroalkylkette mehrere (NR⁴)-Gruppen, so können die von zwei verschiedenen (NR⁴)-Gruppierungen stammenden Reste R⁴ gemeinsam mit den Stickstoffatomen einen 5- bis 6-gliedrigen Ring bilden, so daß die Alkyl- bzw. Heteroalkylgruppe von Imidazolinyl- oder bevorzugt Piperazinylgruppen unterbrochen wird.

Mindestens einer der Reste R² und R³ trägt zusätzlich endständig eine funktionelle Gruppe F¹ bzw. F², die Elektronendonoreigenschaften haben und daher in der Lage sind, sich an die Kationen Q gegebenenfalls koordinativ zu binden. Dies bewirkt eine Stabilisierung der Übergangsmetallkomplexe, insbesondere dann, wenn Q für ein Übergangsmetallkation der Oxidationsstufe I oder für ein Komplexkation der allgemeinen Formel V oder VII steht.

Bevorzugt werden als funktionelle Gruppen F¹ und F² die Gruppen Alkenyl-, Alkinyl-, C₅- bis C₁₂-Cycloalkadienyl-, Nitril-, Amino-, C₁- bis C₄-Mono- und Dialkylamino, C₁- bis C₄-Acyl-, Hydroxyl-, C₁- bis C₄-Alkoxy-, Aryloxy-, C₁- bis C₄-Alkylsulfido-, Arylsulfido-, C₁- bis C₄-Dialkyl- und Diaryl-phosphido und Carboxylato- verwendet.

Kennzeichnend und erfindungswesentlich an den neuen Übergangsmetallkomplexen der allgemeinen Formel I und ursächlich für ihre katalytischen und chemischen Eigenschaften, wie Stabilität und Löslichkeit, ist deren Komponente A, welche selbst ein Übergangsmetallkomplex ist.

Die Anwesenheit der wie beschrieben zusammengesetzten Komplexe A in den Übergangsmetallkomplexen I führt zu einer Reihe von Vorteilen. So sind die Komplexe A in der Lage, über die Sauerstoffatome der Phosphon-, Phosphin-, Arson- und Arsinsäureesterliganden als Mono- oder insbesondere als Di- oder Tripodligand für andere Metallionen oder kationische Metallkomplexe Q zu fungieren. Der Komplex A dient somit gleichzeitig als Ligand und Gegenion für andere Komplexe oder Metallionen Q, welche folglich durch den Komplex A stabilisiert werden. Diese stabilisierende Wirkung der Komplexe A kommt insbesondere in den Komplexen I mit Komplexen Q der allgemeinen Formeln V und VII zur Geltung, welche sich durch eine außergewöhnliche chemische Stabilität auszeichnen.

Weiterhin ermöglichen es die im Komplex A enthaltenen funktionellen Gruppen F aufgrund ihrer Elektronendonoreigenschaften mit dem Komplex A koordinierte Metallkomplex-Fragmente so zu stabilisieren, daß diese ihre katalytische Aktivität sowohl unter den Reaktionsbedingungen als auch unter den Bedingungen der Aufarbeitung des Reaktionsgemisches bewahren.

Außerdem hat man es, je nachdem wie man die Zusammensetzung der Reste R² und R³ im Komplex A wählt, in der Hand, die Löslichkeit der diesen Komplex enthaltenden Übergangsmetallkomplexe zu beeinflussen. Da diese Komplexverbindungen wirksame Katalysatoren sind, kann man somit die Löslichkeit dieser Katalysatoren maßgeschneidert den jeweiligen Erfordernissen der zu katalysierenden Umsetzungen anpassen. Dies ermöglicht den Einsatz dieser Homogenkatalysatoren bei einer Vielzahl von Umsetzungen, wie beispielsweise der Hydrierung von Alkenen, Alkinen und Azomethinen und der Hydroformylierung von Alkenen, ohne daß die eingangs beschriebenen Probleme bei der Abtrennung des Katalysators auftreten. Je nach Art und Anzahl der Liganden können die Komplexe A neutral (A = Komplex I) oder einfach oder zweifach negativ geladen sein. Besonders günstige Eigenschaften haben jedoch die Komplexe I, in denen A einfach negativ geladen und mit einer Komponente Q verbunden ist. Beispielhafte Vertreter der Neutralkomplexe A sind die Verbindungen (1) und (2). 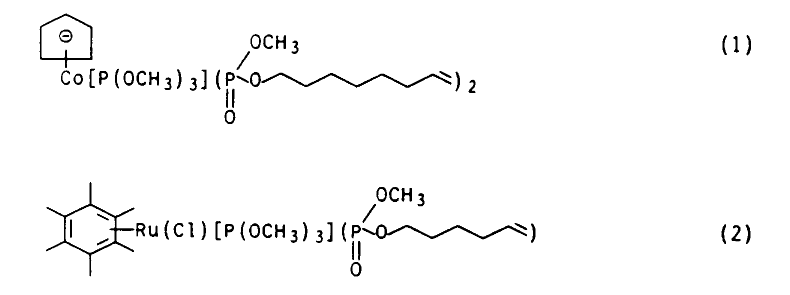
Je nachdem mit welchen Kationäquivalenten Q der Übergangsmetallkomplex A in den Übergangsmetallkomplexen der allgemeinen Formel I verbunden ist, kann die Bindung zwischen A und Q mehr ionischer (salzartiger) oder koordinativer Art sein. Die Grenze zwischen ionischer und koordinativer Bindung ist fließend. Überwiegend ionische Verbindungen liegen in den Verbindungen der allgemeinen Formel I vor, in denen A mit Protonen, Alkalimetallionen, Erdalkalimetallionen und Oniumionen als Kationäquivalenten Q verbunden ist. Als Oniumionen seien beispielhaft Ammonium-, Tetraalkylammonium-, Arylalkylammonium-, Alkylpyridinium-, Tetraarylarsonium- und Tetraarylphosphonium- oder Tetraalkylphosphoniumionen genannt. Da diese Oniumionen nur die Funktion eines Gegenions haben, können sie beliebig gewählt werden. Dies trifft auch bezüglich ihres Substitutionsmusters zu.

Die Übergangsmetallkomplexe I, worin [Q]⁺ einem Alkalimetall- oder Oniumion entspricht, eignen sich in hervorragender Weise zur Herstellung der katalytisch hoch aktiven Komplexe I, in denen Q Komplexen der allgemeinen Formeln V und VII entspricht.

Komplexe I, in denen Q einem Alkalimetall- oder Oniumion entspricht, werden im folgenden zur Vereinfachung als Komplexe A bezeichnet.

Besonders bevorzugte Übergangsmetallkomplexe I sind solche, in denen A ausgewählt ist aus den Übergangsmetallkomplexen der allgemeinen Formel III

[L-Co-B₃]⁻[Q]⁺ III

wobei E der Gruppierung B Phosphor bedeutet,
den Übergangsmetallkomplexen der allgemeinen Formel III, in denen E der Gruppierung B Phosphor bedeutet, c gleich 0 ist und R³ ausgewählt ist aus C₁- bis C₁₆-Alkylen,
den Übergangsmetallkomplexen der allgemeinen Formel IV, 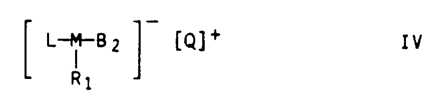
in denen M ausgewählt ist aus Rhodium-, Iridium- und Rutheniumkationen und wobei E der Gruppierung B Phosphor bedeutet sowie den Übergangsmetallkomplexen der allgemeinen Formel IV, in denen M ausgewählt ist aus Rhodium-, Iridium- und Rutheniumkationen, E der Gruppierung B Phosphor bedeutet, c gleich 0 ist und R³ ausgewählt ist aus C₁- bis C₁₆-Alkylen.

Beispielhaft für derartige Komplexe I, in denen A mit Alkalimetall- oder Oniumionen verbunden ist, seien die Verbindungen (3) - (17) aufgeführt. 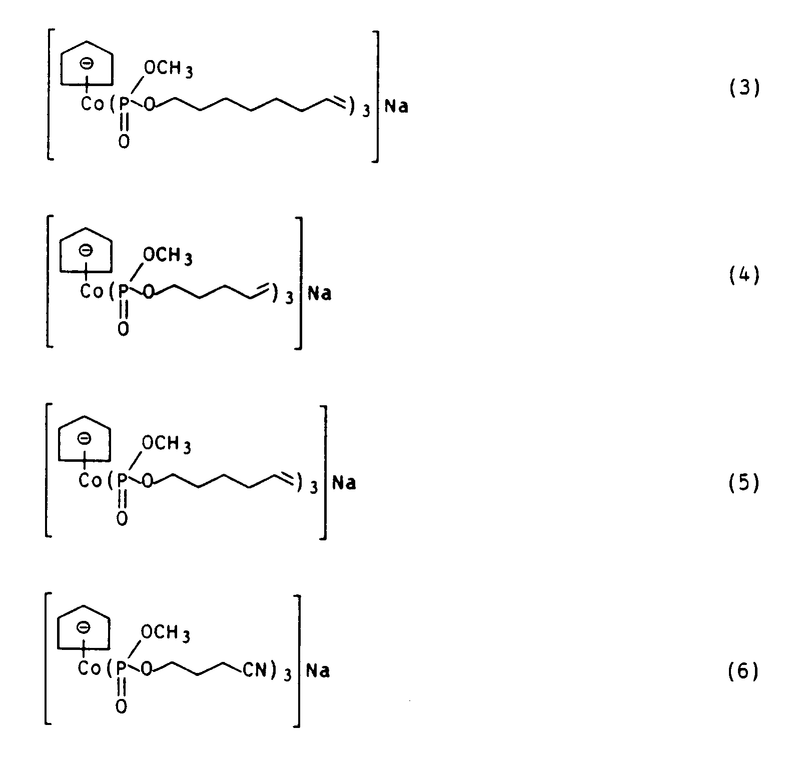 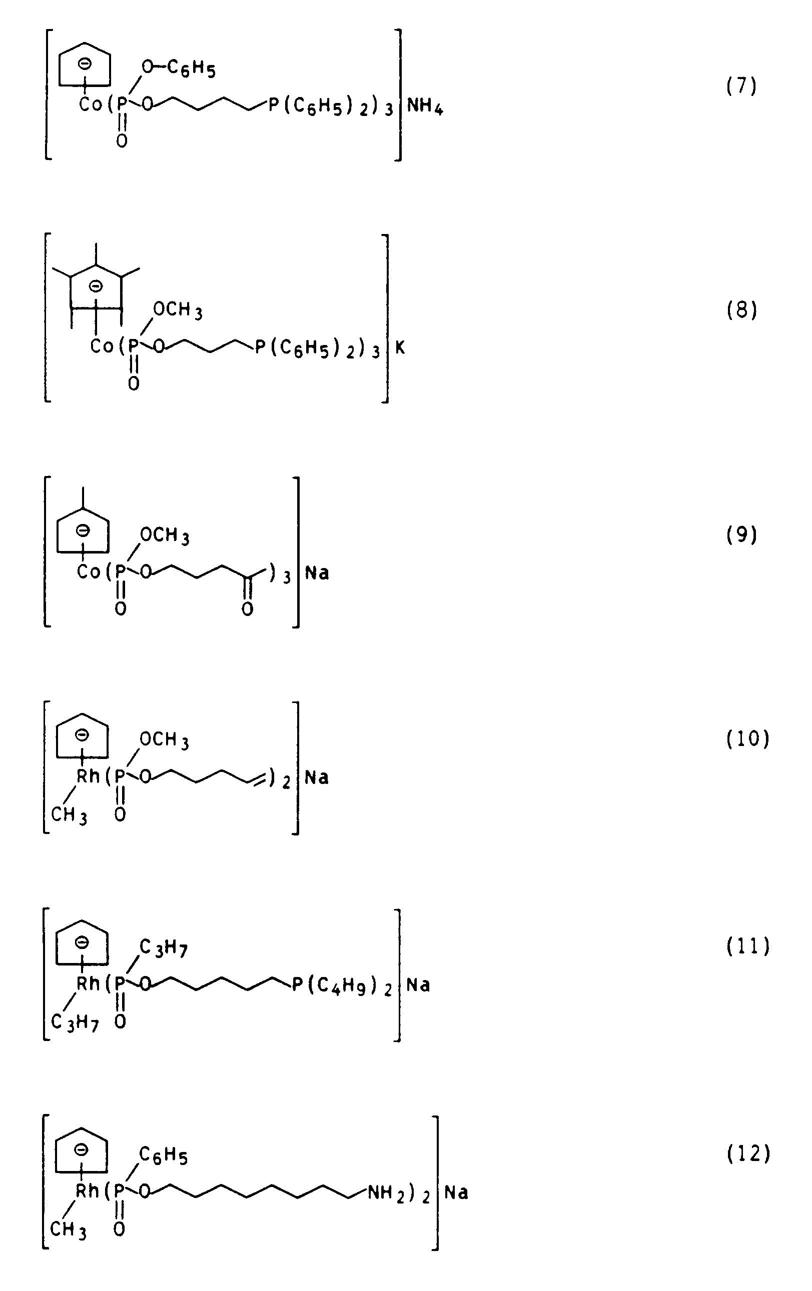 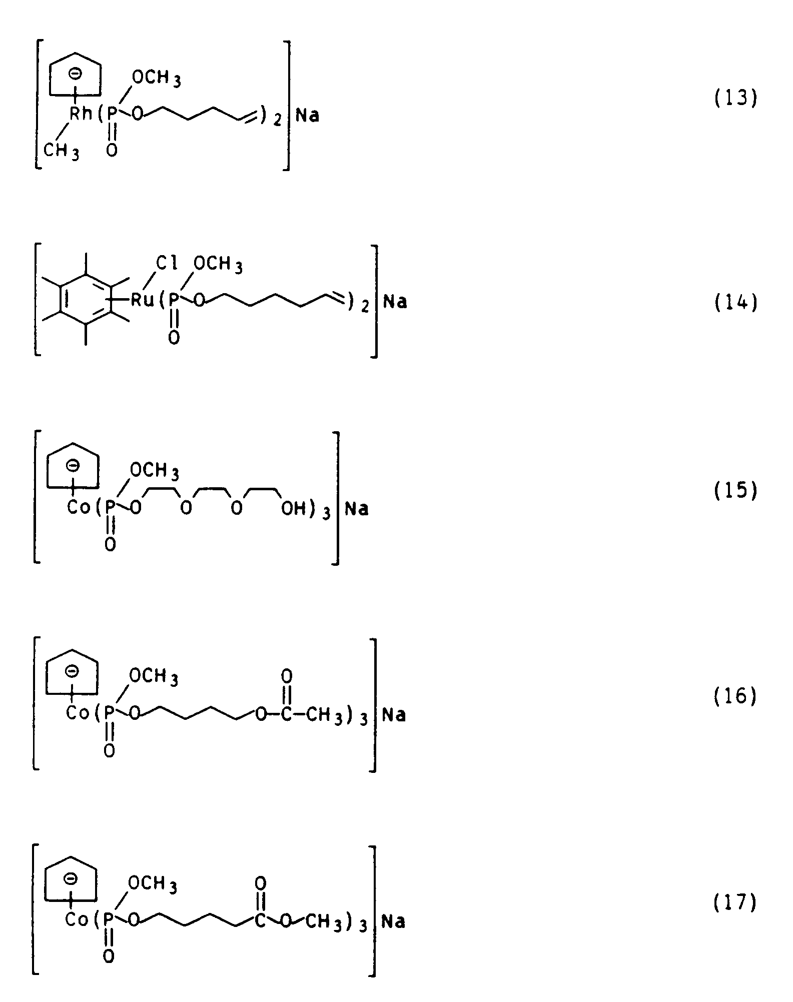
Erstaunlich ist das Löslichkeitsverhalten dieser Verbindungen. So sind beispielsweise die Verbindungen (3), (4), (10) und (14) in Kohlenwasserstoffen sehr gut löslich, während sie in Wasser unlöslich sind, umgekehrt sind z.B. die Verbindungen (9) und (15) in Wasser sehr gut löslich.

Komplexe die Liganden ähnlicher Struktur wie A enthalten, nämlich mit Methyl- und Ethylphosphonaten als Liganden des Zentralions M, jedoch ohne funktionelle Gruppen F¹ und F², sind bereits in der Literatur beschrieben und für ihr starkes Komplexierungsvermögen gegenüber Übergangsmetallkationen bekannt. Diese bekannten Komplexe zeichnen sich durch eine außergewöhnliche Stabilität aus, sind jedoch für katalytische Zwecke wenig geeignet. (vgl.: W. Kläui et al: Angew. Chem. 97, 697 (1985); W. Kläui et al: Chem. Ber. 115, 1922 (1982); W. Kläui et al: J. Organomet. Chem. 331, 317 (1987); W. Kläui: Z. Naturforsch. 34B, 1403 (1979)).

Besonders bemerkenswert ist die chemische und thermische Stabilität der Komplexe A, insbesondere die der Cobaltkomplexe. Diese sind bis zu Temperaturen von 250°C stabil, werden in Lösung als auch im festen Zustand durch Luftsauerstoff nicht oxidiert und zersetzen sich auch nicht in konzentrierten Säuren, wie Salzsäure, sofern die funktionellen Gruppen F unter diesen Bedingungen nicht reagieren.

Ein weiterer Vorteil ist die leichte Zugänglichkeit der Komplexe I. Im allgemeinen geht man bei deren Synthese so vor, daß man zunächst die Komplexe A darstellt. Zur Darstellung der Komplexe A, mit Cobalt als Zentralion, geht man zweckmäßigerweise von Cobalt-Carbonyl-Komplexen wie [LCo(CO)₂] oder [LCo(CO)I₂] aus, in denen L die vorgenannte Bedeutung hat.

In einfachen Ligandenaustauschreaktionen mit tertiären Phosphiten lassen sich daraus die entsprechenden Cobalt-Phosphit-Komplexe darstellen (vgl. Schleman et al.: J. Organomet. Chem. 323, 103 (1987); W. Kläui et al: Chem. Ber. 115, 1922 (1982)). Diese Phosphitkomplexe werden in Gegenwart von Halogenidionen, vorzugsweise Iodidionen, Alkylhalogeniden, vorzugsweise Alkyliodiden oder Pseudohalogenidionen, vorzugsweise Cyanidionen, in einer Michaelis-Arbuzov-Reaktion in die entsprechenden Phosphonsäureester-Komplexe umgewandelt. Je nach den angewandten Reaktionsbedingungen (Menge der Reagenzien, Reaktionstemperatur) lassen sich bei diesen Reaktionen sowohl das Ausmaß des Ligandenaustauschs als auch das Ausmaß der Phosphit-Phosphonsäure-Umwandlung steuern, so daß letztendlich Komplexe mit 1, 2 oder 3 Phosphonsäureesterliganden und 2, 1 bzw. 0 Liganden R¹ gewonnen werden können.

Zur Darstellung der Komplexe 1, in denen A ein Rhodium(I)komplex ist, wird bevorzugt von Rhodium(I)olefinverbindungen wie Cyclooctadien(COD)rhodium(I)chlorid {(COD)RhCl}₂ ausgegangen. Durch Ligandenaustausch lassen sich daraus mit tertiären Phosphiten die entsprechenden Rhodium(I)-Phosphit-Komplexe gemäß Gleichung (a) darstellen, worin die Gruppen R gleich oder verschieden sein können und in ihrer Zusammensetzung, der für die Gruppen R² und R³ angegebenen Zusammensetzung entsprechen.

((COD)RhCl)₂ + 4P(OR)₃ → 2Rh(Cl)[P(OR)₃]₂ (a)

Der gewünschte Ligand L kann dann durch Austausch des Chloridliganden, beispielsweise mittels Cyclopentadienylthallium, in den Komplex eingeführt werden. Der so erhaltene Phosphit-Komplex kann dann wieder über eine Michaelis-Arbuzov-Reaktion in den Phosphonat-Komplex A umgewandelt werden (vgl.: H. Werner et al: Z. anorg. allg. Chem. 458, 301 (1979)).

Die Komplexe I, in denen A Iridium oder Ruthenium als Zentralion M enthalt, werden auf analoge Weise synthetisiert.

Die Herstellung der als Ausgangsmaterial verwendeten Metallkomplexe ist bekannt und kann den zitierten Literaturstellen oder Lehrbüchern wie Brauer, Handbuch der Präparativen Anorganischen Chemie, Enke, 3. Auflage, Band 3, Stuttgart, 1981, entnommen werden.

Tertiäre Phosphite, die mit gleichen oder verschiedenen Alkoholen verestert sind, werden nach den Methoden aus Houben-Weyl, Methoden der Organischen Chemie, Band XII/2, S. 53 bis 73, Thieme, Stuttgart, 1964, gewonnen.

Die Darstellung von Komplexen I, in denen A Phosphinatliganden enthält, wird auf die gleiche Weise, nämlich durch Ligandenaustauschreaktion mit Phosphinitestern und anschließende Michaelis-Arbuzov-Reaktion, bewerkstelligt. Die verwendeten Phosphinitester werden nach Literaturmethoden hergestellt (s.: Houben-Weyl, Methoden der Organischen Chemie, Band XII/1, S. 247 - 261, Thieme, Stuttgart, 1964).

Analoges gilt für die Darstellung der Komplexe I, in denen A Arsonat- oder Arsinat-Liganden enthält.

Die erfindungsgemäßen Komplexe A verhalten sich, je nachdem ob sie 2 oder 3 Phosphonat- und/oder Phosphinat- oder Arsonat- und/oder Arsinatgruppen enthalten, wie zwei- oder dreizähnige Sauerstoffliganden. Als dreizähnige Sauerstoffliganden sind die Komplexe A als 6-Elektronen-Donoren formal isoelektronisch mit dem Cyclopentadienylliganden und wie dieser in der Lage, mit fast allen Übergangsmetallionen, welche in den verschiedensten Oxidationsstufen vorliegen können, Komplexverbindungen zu bilden. Bemerkenswerterweise können die Komplexe A als dreizähnige Sauerstoffliganden mit niederwertigen metallorganischen Komplexfragmenten Komplexe I bilden, die oftmals stabiler sind als die entsprechenden Komplexe mit dem Cyclopentadienylliganden.

Bei den zweizähnigen oder einzähnigen Sauerstoffliganden A übernehmen die funktionellen Gruppen F¹ und F² der Gruppen R² und/oder R³ die Aufgabe als Elektronendonor und helfen auf diese Weise, die Komplexe I von A mit metallorganischen Komplexfragmenten Q der allgemeinen Formel V und VII zu stabilisieren.

Übergangsmetallkomplexe I in denen [Q]⁺ ein Komplexkation des Typs V

[M^{Q}Z]^{x⊕} V

bedeutet, worin
X einen Wert von 0, 1 oder 2 hat,
M^{Q} für Rhodium, Iridium, Ruthenium, Chrom, Wolfram, Molybdän, Mangan, Rhenium oder Kupfer steht,
Z für einen der zweizähnigen Liganden Cycloheptatrien, C₅- bis C₁₂-Cycloalkadien, C₄- bis C₈-Alkadien oder einen C₂- bis C₆-Alkylendiphosphido-Liganden der allgemeinen Formel VI

R⁶-P-(CH₂)₁₋₆-P-R⁶ VI,

worin R⁶ einen C₁- bis C₄-Alkyl- und/oder Phenylrest bedeutet, und/oder für 1 bis 3 gleiche oder verschiedene der einzähnigen Liganden Carbonyl, Nitrosyl, Chlor, Brom, Iod, Nitril, Isonitril, C₁- bis C₄-Alkyl, C₁- bis C₄-Acyl, C₁- bis C₄-Alken, C₁- bis C₄-Alkin, C₁- bis C₄-Trialkylphosphino, Triarylphosphino- und/oder Carbin-Liganden steht, und wobei es als selbstverständlich vorausgesetzt wird, daß die Anzahl der Liganden Z, abhängig ist von der Koordinationszahl der Metalle M^{Q}, werden zweckmäßigerweise aus den Übergangsmetallkomplexen I, in denen Q einem Alkalimetall- oder Oniumion entspricht, hergestellt.

Beispielhaft seien an dieser Stelle einige Synthesewege zur Herstellung der Komplexe I, worin Q für einen Komplex der allgemeinen Formel V steht, angegeben.

Als besonders geeignete Ausgangsmaterialien zur Herstellung der Komplexe I, in denen M^{Q} Wolfram, Chrom oder Molybdän ist, erweisen sich die Carbonylkomplexe dieser Metalle. Daraus lassen sich durch einfachen Ligandenaustausch mit dem Liganden A die entsprechenden Komplexe I mit Q der allgemeinen Formel V gewinnen.

So läßt sich beispielsweise die Verbindung hapto 7-Cycloheptatrienyl-tricarbonyl-wolfram [(h⁷-C₇H₇)W(CO)₃]⁺ mit den Salzen der Komplexe [A]⁻ zu Verbindungen des Typs ([A][W(CO)₂(h³-C₇H₇)]) umsetzen. Analog verläuft die Umsetzung der Komplexe A mit Carbinkomplexen wie trans-[BrMo(CO)₄(Phenylcarbin)] zu Komplexen VII des Typs [A][Mo(CO)₂(Phenylcarbin)]. Natürlich läßt sich diese Reaktion auch mit anderen Carbinliganden als Phenylcarbin durchführen.

Die durch Ligandenaustausch mit den Übergangsmetallkomplexen A aus den Carbonylkomplexen des Wolfram, Chrom und Molybdän der formalen Oxidationsstufe 0 herstellbaren anionischen Komplexe, können ebenfalls vorteilhaft zur Herstellung der Komplexe I mit Q der allgemeinen Formel V genutzt werden. So erhält man beispielsweise durch Umsetzung der Tri-acetronitril-tricarbonyl Komplexe [(CH₃CN)₃M^{Q}(CO)₃] des Wolframs, Chroms und Molybdäns mit den Komplexen A anionische Komplexe des Typs ([A][M^{Q}(CO)₃])⁻. Werden die Lösungen dieser Verbindungen mit verdünnten Mineralsäuren oder organischen Säuren angesäuert, so erhält man die konjugierten Säuren ([A][M^{Q}(CO)₃])⁻H⁺. Diese werden unter Einwirkung von Isopren in die entsprechenden erfindungsgemäßen hapto 3-Dimethylallylkomplexe [A][M^{Q}(CO)₃(h³-dimethylallyl)] umgewandelt.

In den meisten Fällen ist jedoch der Umweg über die konjugierte Säure nicht erforderlich. So liefert zum Beispiel die Umsetzung der Tricarbonylkomplexe ([A][M^{Q}(CO)₃])⁻ des Wolframs, Chroms und Molybdäns mit N-Methyl-N-nitroso-4-toluolsulfonsäureamid die entsprechenden erfindungsgemäßen Nitrosylkomplexe [A][M^{Q}(CO)₃(NO)].

Die anionischen Tricarbonylkomplexe des Wolframs, Chroms und Molybdäns können auch durch chemische Reaktionen in der Ligandensphäre in die erfindungsgemäßen Komplexe I mit Q der allgemeinen Formel V überführt werden. So findet bei der Umsetzung der Salze der Tricarbonylkomplexe ([A][M^{Q}(CO)₃])⁻ des Wolframs, Chroms und Molybdäns mit C₁- bis C₄-Alkyliodiden RI zum einen eine einfache Anlagerung eines Alkylrestes an den Carbonylkomplex statt, wobei die Komplexe I des Typs [A][M^{Q}(CO)₃R] entstehen, gleichzeitig erhält man bei dieser Umsetzung aber auch Dicarbonyl-hapto 2-acyl-komplexe 1 [A][M^{Q}(CO)₂(h²-COR)], in denen ein Carbonylligand mit dem Alkylrest unter Bildung eines Acylliganden reagiert hat. Diese beiden Reaktionsprodukte lassen sich mittels Säulenchromatographie leicht voneinander trennen.

Eine ähnliche chemische Umwandlung in der Ligandensphäre findet bei der Oxidation der Komplexe I [A][M^{Q}(CO)₃(CH₃)] in einer Sauerstoffatmosphäre statt. Dabei bildet sich der entsprechende Dioxo-acetato-Komplex I [A][M^{Q}(O)₂(acetat)].

Bei der Darstellung der Komplexe I, in denen Q der allgemeinen Formel V ein Mangan- oder Rheniumkomplex ist, wird auf analoge Weise vorgegangen. So ergibt die Umsetzung der Komplexe A mit Halogenido-pentacarbonyl-komplexen wie (BrM^{Q}(CO)₅] des Mangans und des Rheniums durch Ligandenaustausch die erfindungsgemäßen Komplexe I [A][M^{Q}(CO)₃].

Zur Herstellung der Komplexe I mit M^{Q} gleich Rhodium, Iridium und Ruthenium geht man im allgemeinen von den Halogeniden dieser Metalle aus. So führt die Umsetzung der Komplexe A mit Rutheniumtrichlorid unter einer Kohlenmonoxid-Atmosphäre zu Komplexen I des Typs [A][Ru(CO)₂Cl]. Die entsprechenden Rhodium- und Iridiumkomplexe können auf die gleiche Art und Weise erhalten werden. Durch Ligandenaustausch können aus diesen Verbindungen weitere Komplexe I darstellt werden. So ergibt zum Beispiel die Umsetzung von [A][Ru(CO)₂Cl] mit Triphenylphosphin (PPh₃) ein Gemisch bestehend aus [A][Ru(PPh₃)(CO)Cl] und ([A][Ru(PPh₃)(CO)₂]Cl), woraus die Einzelverbindungen mittels Säulenchromatographie isoliert werden können.

Natürlich können die Komplexe I des Rhodiums, Rutheniums und Iridiums auch durch Umsetzung der Komplexe A mit Komplexverbindungen dieser Metalle erhalten werden. So gelangt man beispielsweise ausgehend vom zweikernigen Iridium-cyclooctadien(COD)-dichloro-Komplex [Ir(COD)Cl]₂ bzw. vom zweikernigen Iridium-diethyleno-chloro-Komplex [Ir(CH₂=CH₂)₂Cl]₂ zu den Komplexen I des Typs [A][Ir(COD)] bzw. [A][Ir(CH₂=CH₂)₂] und die Umsetzung des zweikernigen Rhodiumkomplexes [Rh(CO)₂Cl]₂ mit dem Komplex A führt zu den Dicarbonylkomplexen I [A][Rh(CO)₂]. Diese stehen wiederum, in Abhängigkeit von der Temperatur und dem Kohlenmonoxid-Partialdruck über der Verbindung oder deren Lösungen, im Gleichgewicht mit den Monocarbonylkomplexen I [A][Rh(CO)]. Alle diese Komplexe I lassen sich sowohl mit Iridium, Ruthenium als auch mit Rhodium als Zentralatom M^{Q} darstellen. Will man die Monocarbonylkomplexe selektiv erhalten, so führt man die Kohlenmonoxidabspaltung vorteilhaft photochemisch durch Bestrahlen der Dicarbonylkomplexe mit UV-Licht aus.

Zur Synthese der Komplexe I mit Kupfer als Zentralatom M^{Q} wird vorzugsweise von Kupfer(II)komplexen ([A]₂Cu) ausgegangen, welche leicht durch Umsetzung von Kupfer(II)salzlösungen mit Lösungen der Komplexe A zugänglich sind. In Gegenwart von feinverteiltem metallischem Kupfer unter einer Kohlenmonoxid-Atmosphäre werden diese zu Kupfer(I)monocarbonyl-Komplexen I [A][Cu(CO)] reduziert. Diese können aber auch ausgehend von Tetra-acetonitrilo-Kupfer(I)komplexen [Cu(CH₃CN)₄)⁺ oder anderen Solvat-Komplexen des Kupfers durch Ligandenaustausch mit den Komplexen A unter einer Kohlenmonoxid-Atmosphäre dargestellt werden. Führt man diese Reaktion in Gegenwart von Alkenen oder Alkinen anstelle von Kohlenmonoxid durch, so gelangt man zu den Alken- bzw. Alkin-Kupfer-Komplexen I [A][Cu(alken)] bzw. [A][Cu(alkin)]. Werden bifunktionelle Alkene wie Butadien oder Cyclooctadien in diese Reaktion eingesetzt, so führt dies zur Bildung der zweikernigen Komplexe (18) und (19), 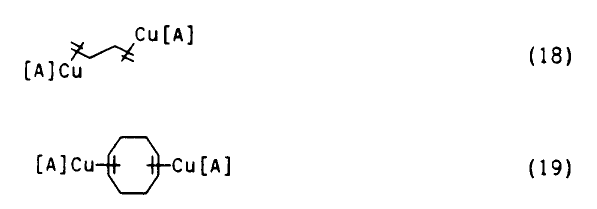
worin der Kupfer-Alken-Komplex einem Kation-Äquivalent Q der allgemeinen Formel VII entspricht.

Zur Darstellung der Komplexe I mit Q der allgemeinen Formel VII und M^{Q} gleich Rhodium, Iridium und Ruthenium wird zweckmäßigerweise von den Dicarbonylkomplexen I des Typs [A][M^{Q}(CO)₂] ausgegangen. Diese dimerisieren in der Wärme unter Kohlenmonoxid-Abspaltung zu Komplexen I wie [A][Rh-µ(CO)₃-Rh][A]. Der griechische Buchstabe µ in der Formel soll dabei kenntlich machen, daß die Carbonylliganden Brückenliganden sind.

Liegen Komplexe I mit unterschiedlichen Zentralatomen M^{Q} Rhodium, Iridium und Ruthenium im Reaktionsgemisch vor, so bilden sich unter diesen Bedingungen auch überbrückte zweikernige Komplexe mit zweierlei Zentralatomen M^{Q} pro Komplex Q. Unter Einwirkung eines hohen Kohlenmonoxid-Partialdrucks können die dimeren Komplexe wieder in die monomeren Komplexe [A][M(CO)₂] zurückverwandelt werden.

Sowohl aus den Komplexen I des Typs [A][M^{Q}(CO)₂] als auch aus den daraus hergestellten Komplexen mit Q der allgemeinen Formel VII können in Gegenwart von Alkadienen, Cycloalkadienen, Alkylisonitrilen und/oder Phosphidoverbindungen durch Ligandenaustauschreaktion Komplexe I erhalten werden, in denen der Komplex Q der allgemeinen Formel VII, verschiedene Brückenliganden pro Komplexmolekül enthält.

Die Komplexe I, in denen Q für ein Übergangsmetallion oder insbesondere für einen Übergangsmetallkomplex der allgemeinen Formeln V und VII steht, haben bemerkenswerte katalytische Eigenschaften. Die Komplexe I, in denen Q für ein Alkalimetall- oder Oniumion steht, sind ebenfalls katalytisch aktiv, doch sind sie in ihrer katalytischen Aktivität den vorgenannten Komplexen I unterlegen.

Komplexe I, in denen Q der allgemeinen Formel V ein Dikation ist, werden beispielsweise durch Umsetzung der Komplexe A mit den dikationischen Metallkomplexen [Ru(hexamethylbenzol)Cl₂] bzw. [Rh(pentamethylcyclopentadienyl)Cl₂] erhalten. Dabei entstehen die Komplexe I der Formeln [A][Ru(hexamethylbenzol]⁺ bzw. [A][Rh(pentamethylcyclopentadienyl]⁺.

Auch bei den Übergangsmetallkomplexe nach Anspruch 1, in denen [Q]⁺ für ein Komplexdikation der allgemeinen Formel VII

[M^{Q}-K-M^{Q}]²⁺ VII

steht und worin K für 1 bis 3 der Brückenliganden Carbonyl, C₄- bis C₆-Alkadienyl, C₈- bis C₁₂-Cycloalkadien, C₁- bis C₄-Alkylisonitril, C₁- bis C₄-Dialkylphosphido, Diarylphosphido und/oder C₁- bis C₆-Alkylendiphosphido-Liganden der allgemeinen Formel VI steht und deren Herstellung oben beschrieben wurde, ist es selbstverständlich, daß die Anzahl der Brückenliganden K von der Koordinationszahl der jeweiligen Metalle M^{Q} im Komplex Q und von der Zahl der Koordinationsstellen der jeweiligen Komplexliganden A abhängt. Die Anzahl der Brückenliganden K kann deshalb nicht größer sein, als daß die Anzahl der Koordinationsstellen im Komplex I, definiert als die Summe der Koordinationsstellen der Komplexliganden A - ohne Berücksichtigung deren funktioneller Gruppen F - und der Koordinationsstellen der Liganden K, die Summe der Koordinationszahlen der jeweiligen Metalle M^{Q} im Komplex VII nicht übersteigt.

Die Komplexe I mit Q der allgemeinen Formeln V und VII werden als Katalysatoren bevorzugt zur Cyclotrimerisierung von Acetylenverbindungen, zur katalytischen Hydrierung ungesättigter Verbindungen wie Alkenen, Alkinen, Aldehyden oder Azomethinen sowie zur Hydroformylierung von C₂- bis C₁₀-Alkenen verwendet.

Dabei kann es vorteilhaft sein, Mischungen dieser Verbindungen untereinander oder Mischungen dieser Verbindungen mit anderen Katalysatoren, Promotoren und/oder Cokatalysatoren einzusetzen. Werden Komplexe mit Q der allgemeinen Formeln V und VII als Katalysatoren verwendet, so erweist es sich oftmals als zweckmäßig, diese Verbindungen in situ in der Reaktionslösung herzustellen.

Bei der spektroskopischen Untersuchung solcher Reaktionslösungen kann man oftmals beobachten, daß mehrere der beschriebenen Komplexe, insbesondere die Carbonylkomplexe Q der allgemeinen Formeln V und VII bei der Hydroformylierungsreaktion, nebeneinander im Gleichgewicht vorliegen.

Dabei kann insbesondere in Abhängigkeit vom Kohlenmonoxid-Partialdruck die Bildung eines bestimmten Komplexes bevorzugt sein. Das Kohlenmonoxid ist in solchen Systemen somit nicht nur als Reaktant sondern auch als Promotor anzusehen.

Durch Verwendung von Cokatalysatoren, beispielsweise Triphenylphosphin, hat man es in der Hand, die Selektivität der Reaktion zu steuern. Da das n/iso-Verhältnis bei der Hydroformylierung auch vom jeweils verwendeten Katalysator abhängt, hat man diesbezüglich durch Verwendung der einzelnen katalytisch aktiven, erfindungsgemäßen Komplexe, deren Mischungen oder deren Mischungen mit Promotoren oder Cokatalysatoren eine weitere Möglichkeit zur Steuerung der Selektivität.

Die erfindungsgemäßen Katalysatoren gelangen im allgemeinen bezüglich der in Lösung befindlichen Reaktanten in einem Molverhältnis von 1:50 bis 1:10000, vorzugsweise von 1:100 bis 1:1000, besonders bevorzugt im Molverhältnis von 1:100 bis 1:800 zur Anwendung.

Bei der Cyclotrimerisierung von Acetylenverbindungen werden Molverhältnisse von 1:100 bis 1:200, bei der Hydrierung ungesättigter Verbindungen und bei der Hydroformylierung von Alkenen Molverhältnisse von 1:500 bis 1:1000 vorgezogen.

Die Cyclotrimerisierung von Acetylenverbindungen wird mit den erfindungsgemäßen Katalysatoren im allgemeinen bei Temperaturen von 80 bis 150°C, unter Atmosphärendruck oder unter erhöhtem Druck vorgenommen. Bevorzugt wird die Cyclotrimerisierung bei Atmosphärendruck und Temperaturen von 100 bis 120°C durchgeführt.

Die Alkene und Alkine können mit den erfindungsgemäßen Katalysatoren bei Raumtemperatur aber auch bei Temperaturen bis zu 200°C hydriert werden. Bevorzugt werden jedoch Temperaturen Von 50 bis 100°C. Die Hydrierung erfolgt normalerweise bei einem Wasserstoffdruck von 10 bis 100 bar. Falls erforderlich, können auch niedrigere oder höhere Drücke angewandt werden. So ist es möglich, bei Anwendung milder Reaktionsbedingungen Alkine nur bis zur Stufe der Alkene zu hydrieren. Sollen Carbonylverbindungen hydriert werden, so können je nach verwendetem Katalysator und zu hydrierender Carbonylverbindung Wasserstoffdrücke bis zu 300 bar erforderlich sein.

Die nachfolgenden Beispiele sollen der weiteren Erläuterung der Erfindung dienen.

### Beispiel 1

### Herstellung der Verbindungen Na[(C₅H₅)Co(P(O)(OCH₃)(O-R³-F²))₃)]

### Allgemeine Arbeitsvorschrift

Zu einer Lösung von 3 Äquivalenten des Phosphorigsäuretriesters P(OCH₃)₂(OR³-F) in Aceton wurde langsam 1 Äquivalent der cobaltverbindung [(C₅H₅)Co(CO)J₂] gegeben.

Die zu Beginn schwarzviolette Lösung wurde unter Kohlenmonoxid-Entwicklung rasch gelbbraun. Es wurde 12 h bei Raumtemperatur gerührt, dann ein Äquivalent Natriumjodid zugefügt und nochmals 12 h gerührt. Das Lösungsmittel wurde abdestilliert und der Rückstand über eine Säule aus Kieselgel der Aktivität III chromatographiert. Dabei wurden zunächst mit einem Eluentengemisch, bestehend aus 2/1 Volumenteilen Dichlormethan/Aceton die entstandenen Nebenprodukte entfernt. Anschließend wurde das Produkt mit einem Eluentengemisch aus 1/2 Volumenteilen Aceton/Methanol von der Säule eluiert. Das Lösungsmittel wurde abdestilliert und das Produkt dabei in reiner Form erhalten.
1.1 Na[(C₅H₅)Co{P(O)(OCH₃)(O-CH₂-CH₂-CH₂-CH=CH₂)}₃]
   Herstellung wie in der allgemeinen Arbeitsvorschrift beschrieben
   - Ansatz:: 4,646 g (26,08 mmol) P(OCH₃)₂(OCH₂-CH₂-CH₂-CH=CH₂)
   3,5 g (8,62 mmol) [(C₅H₅)Co(CO)J₂]
   1,29 g (8,61 mmol) NaJ
   in 50 ml Aceton.
   - Ausbeute:: 4,1 g (76 %)
   ¹H-NMR (80MHz, CDCl₃); δ = 1.51-1.84("p" aus t/t, ³J_{HCCH} = 6.5 Hz, 6H, 3 POCH₂CH₂CH₂CH=CH₂), 2.03-2.29("q" aus d/t, ³J_{HCCH} = 6.6 Hz, 6H, 3 POCH₂CH₂CH₂CH=CH₂), 3.58(virt.q, ³J_{POCH} = 10.4 Hz, 9H, 3 POCH₃), 3.92(sym.m, 6H, 3 POCH₂CH₂-), 4.95(d/m, ³J_{HC=CH(cis)} = 9.9 Hz, 3H, 3-HC=CHH_{cis}), 5.0(s, 5H, C₅H₅), 4.98(d/m, ³J_{HC=CH(trans)} = 17.4 Hz, 3H, 3-HC=CHHₜᵣₐₙₛ), 5.82(t/d/d, ³J_{HCCH} = 6.3 Hz, ³J_{HC=CH(cis)} = 9.9 Hz, ³J_{HC=CH(trans)} = 17.4 Hz, 3H, 3-CH₂CH=CH₂) ³¹P{¹H}-NMR (32MHz, CDCl₃ bei -60°C); δ = 106.8(s)
   IR (Film auf KBr, cm⁻¹): V(C=C) 1640 m, V(P=0) 1160 ss, δ(C=C-H) von C₅H₅) 830 s, δ(P=0) 580 s
1.2 Na[(C₅H₅)Co{P(O)(OCH₃)(O-(CH₂)₆-CH=CH₂)}₃)
   Herstellung wie in der allgemeinen Arbeitsvorschrift beschrieben.
   - Ansatz:: 8,70 g (39,5 mmol) P(OCH₃)₂(O-(CH₂)₆-CH=CH₂)
   5,30 g (13,06 mmol) [(C₅H₅)Co(CO)J₂]
   1,95 g (13,01 mmol) NaJ
   in 50 ml Aceton
   - Ausbeute:: 7,07 g (71 %)
   ¹H-NMR (80MHz, CDCl₃); δ = 1.37-1.85(m, 24H, 3 OCH₂CH₂CH₂CH₂CH₂CH₂CH=CH₂), 1.94-2.09(m, 6H, 3 CH₂CH₂CH=CH2), 3.59(virt.q, ³J_{POCH} = 10.4 HZ, 9H, 3 POCH3), 3,89(sym.m, 6H₄, 3 POCH₂CH₂-), 4.94(d/m), ³J_{HC-CH(cis)} = 9.9 Hz, 3H, 3-HC=CHH_{cis}), 4.98(d/m, ³J_{HC-CH(cis)} = 9.9 Hz, 3H, 3-HC=CHH_{cis}), 4.98(d/m, ³J_{HC=CH(trans)} = 17.3 Hz, 3H, 3-HC=CHHₜᵣₐₙₛ), 5.01(s, 5H, C₅H₅), 5.86(t/d/d, ³J_{HCCH} = 6.5 Hz, ³J_{HC=CH(cis)} = 9.9 Hz, ³J_{HC=CH(trans)} = 17.3 Hz, 3H, 3 -CH₂CH=CH₂) ³¹P{¹H}-NMR (32MHz, CDCl₃ bei -60°C): δ = 106.9(s)
   IR (Film auf KBr, cm⁻¹): ν(C=C) 1640 m, ν(P=O) 1160 ss, δ(C=C-H von C₅H₅) 830 m, δ(P=O) 580 s.
1.3 Na[(C₅H₅)Co{P(O)(OCH₃)(O-CH₂-CH₂-CH₂-CN)}₃]
   Herstellung wie in der allgemeinen Arbeitsvorschrift beschrieben.
   - Ansatz:: 1,859 g (10,5 mmol) P(OCH₃)₂(O-(CH₂-CH₂-CH₂-CN)
   1,209 g (2,96 mmol) [(C₅H₅)Co(CO)J₂]
   0,60 g (4,0 mmol) NaJ
   in 40 ml Aceton
   - Ausbeute:: 1,84 g (98 %)
   ¹H-NMR (80MHz, CDCl₃); δ = 1.96("p" aus t/t, ³J_{HCCH} = 6.5 Hz, 6H, 3 OCH₂CH₂CH₂CN), 2.58(t, ³J_{HCCH} = 6.8 Hz, 6H, 3 CH₂CH₂CH₂CN), 3.60(virt.q, ³J_{POCH} = 10.5 Hz, 9H, 3 POCH3), 3.7-4.2(sym.m, 6H, 3 POCHCH₂-), 5.06(s, 5H, C₅H₅) ³¹P{¹H}-NMR (32MHz, CDCl₃ bei -60°C): δ = 107.8(s)
   IR (Film auf KBr, cm⁻¹): ν(C≡N) 2245 m, ν(P=O) 1160-1135 ss, ν(P-O) 1040-1005 vs, δ(C=C-H von C₅H₅) 830 m, δ(P=O) 570 s
1.4 Na[(C₅H₅)Co{P(O)(OCH₃)(O-(CH₂)₆-CN)}₃)
   Herstellung wie in der allgemeinen Arbeitsvorschrift beschrieben.
   - Ansatz:: 6,824 g (31,13 mmol) P(OCH₃)₂(O-(CH₂)₆-CN)
   3,90 g (9,61 mmol) [(C₅H₅)Co(CO)J₂]
   1,555 g (10,38 mmol) NaJ
   in 100 ml Aceton
   - Ausbeute:: 7,0 g (96 %)
   ¹H-NMR (80MHz, CDCl₃); δ = 1.0-2.0(m, 24H, 3 CH₂CH₂CH₂CH₂CH₂CH₂CN), 2.36(t, ³J_{HCCH} = 6.3 HZ, 6H, 3 -CH₂CH₂CN), 3.57(virt.q, ³J_{POCH} = 9.9 Hz, 9H, 3 POCH3), 3.67-4.0(sym.m, 6H, 3 POCH₂CH₂-), 5.01(s, 5H, C₅H₅) ³¹P{¹H}-NMR (32MHz, CDCl₃ bei -60°C): δ = 108.4(s)
   IR (Film auf KBr, cm⁻¹): ν(C≡N) 2245 w, ν(P=O) 1160 vs, ν(P-O) 1050-1010 vs, δ(C=C-H von C₅H₅) 830 m, δ(P=O) 570 s.
1.5 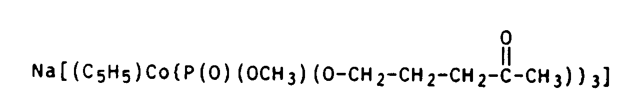 Herstellung wie in der allgemeinen Arbeitsvorschrift beschrieben.
   - Ansatz:: 3,24 g (16,61 mmol) 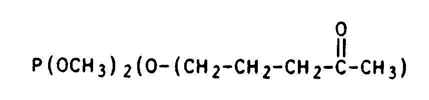 2,247 g (5,53 mmol) [(C₅H₅)Co(CO)J₂]
   0,83 g (5,53 mmol)NaJ
   - Ausbeute:: 2,92 g (77 %)
   ¹H-NMR (80MHz, CDCl₃); 1.86("p", ³J_{JCCH}= 6.7 Hz, 6H, 3 CH₂CH₂CH₂C(O)CH₃), 2.17(s, 9H, 3 CH₂C(O)CH₃), 2.57(t, ³J_{HCCH} = 7.1 Hz, 6H, 3 CH₂CH₂C(O)CH₃), 3.56(virt.q, ³J_{POCH} = 9.8 Hz, 9H, 3 POCH3), 3.90(sym.m aus t/virt.q, 6H, 3 POCH₂CH₂), 5.00(q, ³J_{POCH} = 0.3 Hz, 5H, C₅H5) ³¹P{¹H}-NMR (32MHz, CDCl₃ bei -60°C): δ = 107.1(s)
   IR (Film auf KBr, cm⁻¹): ν(C=0) 1712 vs, ν(P=O) 1165 ss, 1140 s, ν(P-O) 1050-1000 vs, δ(C=C-H von C₅H₅) 830 m, δ(P=O) 570 s

### Beispiel 2

### Herstellung der Verbindungen Na[(C₅H₅)Rh(CH₃){P(O)(OCH₃)(O-R³-F³)}₂]

### Allgemeine Arbeitsvorschrift

Zu einer Lösung von Cyclooctadienrhodiumchlorid [{(COD)RhCl}₂] in 40 ml Dichlormethan wurde unter Rühren bei Raumtemperatur langsam der Phosphorigsäuretriester P(OCH₃)₂(O-R³-F) gegeben. Dabei heilte sich die Farbe der Lösung von orange nach hellgelb auf. Es wurde 2 h gerührt. Danach wurden sämtliche flüchtigen Komponenten des Reaktionsgemisches im Hochvakuum abgezogen. Der Rückstand wurde in 30 ml Tetrahydrofuran aufgenommen und zu dieser Lösung ein geringer Überschuß Cyclopentadienylthallium Tl(C₅H₅) gegeben. Die entstandene Suspension wurde 24 h bei Raumtemperatur gerührt. Anschließend wurde das Lösungsmittel im Vakuum abdestilliert. Der rote Rückstand wurde mit Diethylether über eine Säule aus Kieselgel der Aktivitätsstufe III chromatographiert. Die erste gelbe Fraktion, die eluiert wurde, wurde gesammelt und eingeengt. Sie enthielt die Rhodiumverbindung [(C₅H₅)Rh{P(OCH₃)₂(O-R³-F)}₂]. Diese Verbindung wurde in 50 ml Aceton gelöst. Diese Lösung wurde mit Natriumiodid versetzt und anschließend 6 bis 8 Stunden auf 50°C erwärmt. Der Verlauf der Reaktion wurde NMR-spektroskopisch verfolgt. Nachdem die Reaktion beendet war, wurde das Lösungsmittel entfernt und das Produkt zur Entfernung überschüssigen Natriumiodids mit Toluol extrahiert. Das Extraktionsmittel wurde abdestilliert und man erhielt das reine Produkt.
2.1 Na[(C₅H₅)Rh(CH₃){P(O)(OCH₃)(O-CH₂-CH₂-CH₂-CH=CH₂)}₂]
   Herstellung wie in der allgemeinen Arbeitsvorschrift beschrieben.
   - Ansatz:: 1,242 g (6,97 mmol) P(OCH₃)₂(O-CH₂CH₂CH₂-CH=CH₂)
   0,8 g (1,62 mmol) [{(COD)RhCl}₂]
   1,1 g (4,08 mmol) Tl(C₅H₅)
   1,05 g (7,0 mmol) NaJ
   - Ausbeute:: 1,48 g (86 %)
   ¹H-NMR (80MHz, CDCl₃); 0.63(m, 3H, RhCH₃), 1.10-2.00(m, 4H, 2 OCH₂CH₂CH₂CH=CH₂), 1.98-2.18("q", ³J_{HCCH} = 6.3 Hz, 4H, 2 OCH₂CH₂CH₂CH=CH₂), 3.52(virt.t, ³J_{POCH} = 11.9 Hz, 6H, 2 POCH3), 3.86(sym.m, 4H, 2 POCH₂CH₂-), 4.95(d/m), ³J_{HC-CH} = 9.9 Hz, 2H, 2 -CH=CHH_{cis}), 4.98(d/m, ³J_{HC-CH(trans)} = 17.4 Hz, 2H, 2 -CH=CHHₜᵣₐₙₛ), 5.32(t, ³J_{PRhCH} = 2.2 Hz, 5H, C₅H₅), 5.82(t/d/d, ³J_{HCCH} = 6.4 Hz, ³J_{HC=CH(cis)} = 9.9 Hz, ³J_{HC=CH(trans)} = 17.4 Hz, 2H, 2 -CH₂CH=CH₂) ³¹P{¹H}-NMR (CDCl₃ bei -60°C): δ = 102.6(d, ¹J_{RhP} = 209 Hz)
   IR (Film auf KBr, cm⁻¹): ν(C=C) 1640 m, ν(P=O) 1130 vs, δ(P=O) 570 s
2.2 Na[(C₅H₅)Rh(CH₃){P(O)(OCH₃(O-CH₂-CH₂-CH₂-CN)}₂]
   Herstellung wie in der allgemeinen Arbeitsvorschrift beschrieben.
   - Ansatz:: 2,11 g (11,9 mmol) P(OCH₃)₂(O-CH₂CH₂CH₂-CN)
   1,145 g (2,32 mmol) [{(COD)RhCL}₂]
   1,785 g (6,52 mmol) Tl(C₅H₅)
   1,66 g (11,1 mmol) NaJ
   - Ausbeute:: 1,48 g (70 %)
   ¹H-NMR (80MHz, CDCl₃); δ = 0.64(m, 3H, RhCH₃), 1.98("p" aus t/t, ³J_{HCCH} = 6.1 Hz, 4H, 2 POCH₂CH₂CH₂CN), 2.54(t, ³J_{HCCH} = 6.6 Hz, 4H, 2 POCH₂CH₂CH₂CN), 3.54(virt.t, ³J_{POCH} = 12.1 Hz, 6H, 2 POCH3), 3.83-4.12(m, 4H, 2 POCH₂CH₂-), 5.39(t, ³J_{PRhCH} = 2.3 Hz, 5H, C₅H₅) ³¹P{¹H}-NMR (32MHz, CDCl₃ bei -60°C): δ = 110.1(d, ¹J_{RhP} = 210.6 Hz)
   IR (Film auf KBr, cm⁻¹): ν(C≡N) 2250 m, ν(P=O) 1119 vs, ν(P=O) 1035-1020 vs, δ(P=O) 585 s.

### Beispiel 3

### Darstellung der Verbindungen [A]Rh(µ-CO)₃Rh[A]:(A:[(C₅H₅)Co{P(O)(OCH₃)(O-R³-F²)}₃]⁻)

### Allgemeine Arbeitsvorschrift

Zu einer Lösung von einem Äquivalent der Rhodiumverbindung [{(CO)₂RhCl}₂] in Dichlormethan wurden 2 Äquivalente des Komplexsalzes Na⁺[A]⁻ gegeben und gerührt. Der Ablauf der Reaktion wurde IR-spektroskopisch verfolgt. Nach 30 min hatte sich aus den Ausgangsverbindungen der Komplex ([A][Rh(CO)₂]) gebildet. Nun wurde die Lösung so lange unter Rückfluß erwärmt, bis sich kein Kohlenmonoxid mehr bildete. Dabei entstand durch Decarbonylierung der Komplex [A]Rh(µ-CO)₃Rh[A]. Während der Reaktion beobachtete man im IR-Spektrum des Gemisches, daß die Intensität der Banden bei 2080 und 2000 cm⁻¹, die den Verbindungen ([A][Rh(CO)₂]) zugeordnet wurden, abnahm und eine neue Bande bei 1840 cm⁻¹ entstand, welche den Verbindungen [A]Rh(µ-CO)₃Rh[A] zugeordnet wurde. Nach beendeter Reaktion wurde das Reaktionsgemisch abgekühlt, das Lösungsmittel abdestilliert und das Produkt aus dem Rückstand mit Hexan extrahiert.
3.1 [A]Rh(µ-CO)₃Rh[A]; [A]:[(C₅H₅)Co{P(O)(OCH₃)(O-(CH₂)₆-CH=CH₂)}₃]
   Herstellung wie in der allgemeinen Arbeitsvorschrift beschrieben.
   - Ansatz:: 0,785 g (1,029 mmol) Na[A]
   0,2 g (0,514 mmol) [{(CO)₂RhCl}₂]
   in 50 ml Dichlormethan
   - Ausbeute:: 0,72 g (79 %)
   ¹H-NMR (80MHz, CDCl₃); δ = 3.66(virt.q, ³J_{POCH} = 10.5 Hz, 18H, 6 POCH3), 3.97(sym.m, 12H, 6 POCH₂CH₂-), 4.98(s, 10H, 2 C₅H₅). Die Resonanzsignale der anderen Protonen blieben im Vergleich zur Ausgangsverbindung Na [A] aus Beispiel 1.2 unverändert.
   IR (Film auf KBr, cm⁻¹): ν(CO) 1840 vs, ν(C=C) 1640 w, ν(P=O) 1110 vs, δ(C=C-H von C₅H₅) 830 m, δ(P=O) 590 s.
3.2 [A]Rh(µ-CO)₃Rh[A]; [A]:[(C₅H₅)Co{P(O)(OCH₃)(O-(CH₂)₆-CN)}₃]⁻
   Herstellung wie in der allgemeinen Arbeitsvorschrift beschrieben.
   - Ansatz:: 0,16 g (0,21 mmol) Na[A]
   0,04 g (0,103 mmol) [{(CO)₂RhCl}₂]
   in 20 ml Dichlormethan
   - Ausbeute:: 0,115 g (63 %)
   IR (Film auf KBr, cm⁻¹): ν(C≡N) 2245 w, ν(CO) 1835 vs, ν(P=O) 1110 vs, ν(P-O) 1040-1000 vs, δ(C=C-H von C₅H₅) 830 m, δ(P=O) 590 s.

### Beispiel 4

### Herstellungsweise der Verbindungen [A]Rh(CO); [A]: [(C₅H₅)Co{P(O)(OCH₃)(O-R³-F)}₃]

### Vorschrift (a)

(a) Eine Lösung der Verbindung [A]Rh(µ-CO)₃Rh[A] wurde in Toluol 4 h unter Rückfluß erhitzt und der Ablauf der Reaktion durch IR-Spektroskopie verfolgt. Nachdem die Edukt-Bande bei 1840 cm⁻¹ im IR-Spektrum verschwunden war, wurde die Reaktionsmischung abgekühlt. Das Lösungsmittel wurde abdestilliert und das Reaktionsgemisch über eine Kieselgelsäule chromatographiert. Dabei wurden zunächst die Nebenprodukte mit Aceton, anschließend das Produkt mit Aceton/Methanol im Volumenverhältnis von 1/1 eluiert.

### Vorschrift (b)

(b) Eine Lösung von [A]Rh(µ-CO)₃Rh[A] in Toluol wurde bei Raumtemperatur 6 h lang mit einer Quecksilber-Hochdrucklampe (Philips HPK 125) bestrahlt. Das erhaltene Reaktionsgemisch wurde wie unter 4a) beschrieben aufgearbeitet.

4.1 [A]RhCO; [A]:[(C₅H₅)Co{P(O)(OCH₃)(O-(CH₂)₃-CH=CH₂)}₃]
   Herstellung nach Vorschrift (a):
   - Ansatz:: 0,150 g [A]Rh(µCO)₃Rh[A]
   - Ausbeute:: 30 %
   Herstellung nach Vorschrift (b):
   - Ansatz:: 0,150 g [A]Rh(µCO)₃Rh[A]
   - Ausbeute:: 70 %
4.2 [A]RhCO; [A]:[(C₅H₅)Co{P(O)(OCH₃)(O-(CH₂)₆-CH=CH₂)}₃]
   Herstellung nach Vorschrift (b):
   - Ansatz:: 0,2 g [A]Rh(µCO)₃Rh[A]
   - Ausbeute:: 60 %

### Beispiel 5

### Synthese von [A][Rh(CO)₂]; [A]:[(C₅H₅)Rh(CH₃){P(O)(OCH₃)(O-(CH₂)₃-CH=CH₂)}₃]⁻

0,06 g (0,15 mmol) [{(CO)₂RhCl}₂] und 0,164 g (0,3 mmol) Na[A] wurden in 10 ml Dichlormethan gelöst. Nach 30 min war die Reaktion beendet (IR-Kontrolle). Die Lösung wurde vom ausgefallenen Kochsalz abfiltriert und eingeengt. Das Produkt, ein gelbes öl, entstand in 95 % Ausbeute.

In Lösung bildete sich beim Stehenlassen aus dem Dicarbonyl-Komplex([A][Rh(CO)₂]) langsam der rote Monocarbonyl-Komplex([A][Rh(CO)]);, der eine charakteristische IR-Bande bei 1995 cm⁻¹ besitzt. Durch Einleiten von Kohlenmonoxid konnte ([A][Rh(CO)] wieder in den Dicarbonyl-Komplex zurückverwandelt werden.

### ARh(CO)₂, ([A]⁻ = [(C₅H₅)Rh(CH₃){P(O)(OCH₃)(OCH₂CH₂CH₂CH=CH₂}₂]⁻):

¹H-NMR (80 MHz, C₆D₆); 1.29-1.39(sym.m, RhCH₃), 3.33-3.60(virt.m, 6H, 2 POCH₃), 3.70-4.10(sym.m, 4H, 2 POCH₂CH₂-), 5.15(t, ³J_{PRhCH} = 2.5 Hz, 5H, C₅H₅). Die Resonanzsignale der anderen Protonen blieben im Vergleich zur Ausgangsverbindung Na[A] aus Beispiel 2.1. unverändert.

### Beispiel 6

### Cyclotrimerisierung von Acetylendicarbonsäuredimethylester

2,42 g (17,16 mmol) Acetylendicarbonsäuredimethylester, gelöst in 20 ml Toluol, wurden in einem Schlenkkolben mit Rückflußkühler unter Rühren auf 120°C erhitzt. Zu dieser Lösung wurden 0,1 g (0,145 mmol) [(C₅H₅)Co{P(O)(OCH₃)(O-(CH₂)₃-CH=CH₂)}₃]Rh(CO), gelöst in 5 ml Toluol, als Katalysator zugegeben. Die Änderung der Eduktkonzentration wurde gaschromatographisch verfolgt. Nach 24 h Reaktionszeit betrug die Ausbeute an Mellithsäurehexamethylester 51 %.

### Beispiel 7

### Hydrierung von Cyclohexen

Zur in situ-Darstellung des Katalysators wurden 0,07 g (0,1 mmol) 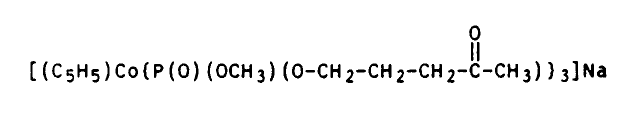
und 0,02 g (0,05 mmol) {(CO)₂RhCl}₂ in 10 ml Dichlormethan gelöst. Nach 15 min wurde das ausgefallene Kochsalz abfiltriert und das Filtrat als Katalysator eingesetzt.

Diese Katalysatorlösung wurde zusammen mit der 1000fachen Menge Cyclohexen (8,11 g; 0,1 mol) in einen 100 ml Edelstahlautoklaven eingefüllt. Anschließend wurde Wasserstoff bis Zu einem Druck von 40 bar in den Autoklaven eingepreßt. Der Autoklav wurde 2 h auf 60°C erwärmt, wobei die Reaktionsmischung gerührt wurde. Anschließend wurde der Autoklav abgekühlt und entspannt und die Reaktionsmischung destillativ aufgearbeitet. Ausbeute: 75 %

### Beispiel 8.1.

### Hydroformylierung von Alkenen

Eine Lösung von 0,1 mmol des Katalysators [(C₅H₅)Co{P(O)(OCH₃)-(O-CH₂-CH₂-CH₂-CH=CH₂)}₃]Rh(CO) in 10 ml Tetrahydrofuran wurde in einem 100 ml Edelstahlautoklaven mit 7 bar Propylen unter Druck gesetzt. Anschließend wurden 20 bar Wasserstoff und 20 bar Kohlenmonoxid aufgepreßt. Die Reaktionsmischung wurde auf 120°C erhitzt und der Druckabfall beobachtet. Die Reaktion war nach 16 h beendet. Die Ausbeute an n- und i-Butyraldehyd betrug 71 %, das n/i-Verhältnis 0,56.

### Beispiel 8.2

Der Versuch aus Beispiel 8.1. wurde wiederholt, jedoch wurde zusätzlich 1 mmol Triphenylphosphin zur Reaktionsmischung gegeben. Nach 16 h Reaktionszeit betrug die Ausbeute an n- und i-Butyraldehyd 99 %, das n/i-Verhältnis hatte sich auf einen Wert von 1,26 verändert.

### Beispiel 8.3

Der Versuch aus Beispiel 8.1. wurde wiederholt, jedoch wurde als Katalysator die Verbindung [(C₅H₅)Co{P(O)(OCH₃)(O-CH₂-CH₂-CH₂-CN)}₃]Rh(CO)₂ eingesetzt und zusätzlich 2,7 mmol Triphenylphosphin zur Reaktionsmischung gegeben. Die Ausbeute an n- und i-Butyraldehyd betrug nach 16 h 99 %. Das n/i-Verhältnis hatte sich unter diesen Bedingungen auf einen Wert von 2,01 erhöht.

### Beispiel 8.4 (Vergleichsbeispiel)

Dieser Versuch wurde ausgeführt, um die Überlegenheit der katalytischen Eigenschaften der erfindungsgemäßen Verbindungen gegenüber den literaturbekannten Komplexverbindungen ohne Funktionalität F in der Phosphonatestergruppe zu demonstrieren. Der Versuch wurde wie in Beispiel 8.1. durchgeführt, jedoch wurde als Katalysator die Verbindung [(C₅H₅)Co{P(O)(OCH₃)₂}₃]Rh(CO) verwendet. Nach 72 h Reaktionszeit betrug die Ausbeute an n- und i-Butyraldehyd nur 8 %.

## Patentansprüche

1. Übergangsmetallkomplexe der allgemeinen Formel I
[A]^{x-} [Q]⁺_{X} I
in der Q für das Äquivalent eines Kations steht, x einen Wert von 0 bis 2 hat und A einem Übergangsmetallkomplex der allgemeinen Formel 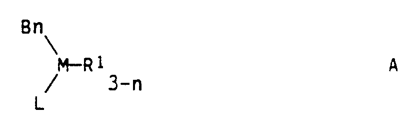 entspricht, in welcher die Variablen folgende Bedeutung haben:
n 1 bis 3
M positiv geladenes Cobalt, Rhodium, Iridium oder Ruthenium
B gleiche oder verschiedene Gruppen der allgemeinen Formel II 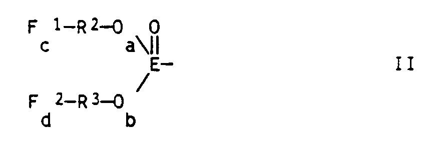 in der die Variablen wie folgt definiert sind:
E Phosphor oder Arsen,
O Sauerstoff,
R², R³ C₁- bis C₂₀-Alkylengruppen, die ihrerseits bis zu zwei Cycloalkyl-, Heterocycloalkyl-, Aryl- oder Heteroarylgruppen mit insgesamt bis zu 15 Ringatomen tragen können und wobei die Reste R² und R³ durch Sauerstoff, Schwefel oder (-NR⁴)-Gruppen unterbrochen sein können, wobei R⁴ Wasserstoff oder C₁- bis C₄-Alkyl bedeutet und wobei die Heteroatome bzw. Heteroatomgruppierungen jeweils durch mindestens 2 Kohlenstoffatome getrennt sind und wobei die von zwei verschiedenen (-NR⁴)Gruppierungen stammenden Reste R⁴ auch miteinander zu einem 5- oder 6-gliedrigen Ring verbunden sein können,
F¹, F² Alkenyl, Alkinyl, C₅- bis C₁₂-Cycloalkadienyl-, Nitril-, Amino-, C₁- bis C₄-Mono- und Dialkylamino, C₁- bis C₄-Acyl-, Hydroxyl-, C₁- bis C₄-Alkoxy-, Aryloxy-, C₁- bis C₄-Alkylsulfido-, Arylsulfido, C₁- bis C₄-Dialkyl- und Diarylphosphido und/oder Carboxylato sind, und falls c oder d gleich 0 sind, für Wasserstoff stehen,
a - d 0 oder 1, mit der Maßgabe, daß a+b>0 und c+d>0 sind
L 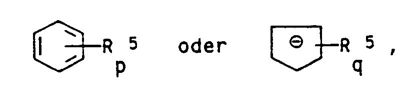 wobei R⁵ für gleiche oder verschiedene Reste aus der Gruppe C₁- bis C₄-Alkyl oder Phenyl steht, p eine der ganzen Zahlen 0 bis 6 und q eine der ganzen Zahlen 0 bis 5 bedeutet,
R¹ Fluor, Chlor, Brom, Iod, Cyanid, Isocyanid, Cyanat, Isocyanat, Thiocyanat, Isothiocyanat, C₁- bis C₄-Alkyl, C₁- bis C₄-Trialkyl- oder Triarylphosphit.

2. Übergangsmetallkomplexe nach Anspruch 1, in dem Q für Lithium, Natrium, Kalium, Silber, Kupfer, Rhodium, Iridium, Ruthenium, Calcium, Magnesium oder ein Ammonium- oder Phosphoniumkation steht.

3. Übergangsmetallkomplexe nach Anspruch 1, der allgemeinen Formel III
[L-Co-B₃]⁻[Q]⁺ III
wobei E der Gruppierung II Phosphor bedeutet.

4. Übergangsmetallkomplexe nach Anspruch 1, der allgemeinen Formel IV, 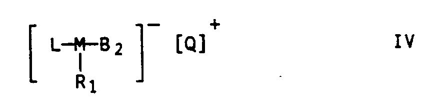 in denen M ausgewählt ist aus Rhodium, Iridium und Rutheniumkationen und wobei E der Gruppierung B Phosphor bedeutet.

5. Übergangsmetallkomplexe nach den Ansprüchen 1, 3 und 4, worin c gleich 0 ist und R³ ausgewählt ist aus C₁- bis C₁₆-Alkylen.

6. Übergangsmetallkomplexe nach Anspruch 1 der allgemeinen Formel I, in denen [Q]⁺_{X} ein Komplexkation des Typs V
[M^{Q}Z]^{x⊕} V
bedeutet, worin
X einen Wert von 1 oder 2 hat,
M^{Q} für Rhodium, Iridium, Ruthenium, Chrom, Wolfram, Molybdän, Mangan, Rhenium oder Kupfer steht,
Z für einen der mehrzähnigen Liganden C₇-Cycloalkatrien, C₅- bis C₁₂-Cycloalkadien, C₄- bis C₈-Alkadien, Aren oder einen C₂- bis C₆-Alkylendiphosphido-Liganden der allgemeinen Formel VI
R⁶-P-(CH₂)₁₋₆-P-R⁶ VI,
worin R⁶ einen C₁- bis C₄-Alkyl- und/oder Phenylrest bedeutet, und/oder für 1 bis 3 gleiche oder verschiedene der einzähnigen Liganden Carbonyl, Nitrosyl, Chlor, Brom, Iod, Nitril, Isonitril, C₁- bis C₄-Alkyl, C₁- bis C₄-Acyl, C₁- bis C₄-Alken, C₁- bis C₄-Alkin, C₁- bis C₄-Trialkylphosphino, Triarylphosphino- und/oder Carbin-Liganden steht.

7. Übergangsmetallkomplexe nach Anspruch 1, in denen [Q]⁺_{X} für ein Komplexdikation der allgemeinen Formel VII
[M^{Q}-K-M^{Q}]²⁺ VII
steht, worin M^{Q} die in Anspruch 6 genannte Bedeutung hat, K für 1 bis 3 der Brückenliganden Carbonyl, C₄- bis C₆-Alkadien, C₈- bis C₁₂-Cycloalkadien, C₁- bis C₄-Alkylisonitril, C₁- bis C₄-Dialkylphosphido, Diarylphosphido und/oder C₁- bis C₆-Alkylendiphosphido-Liganden der allgemeinen Formel VI stehen.

8. Verfahren zur katalytischen Hydrierung ungesättigter Verbindungen, dadurch gekennzeichnet, daß man hierzu Übergangsmetallkomplexe nach den Ansprüchen 1, 3, 4, 5, 6 und 7 als Katalysatoren einsetzt oder diese in situ aus den Übergangsmetallkomplexen nach Anspruch 2 herstellt.

9. Verfahren nach Anspruch 8, dadurch gekennzeichnet, daß man Alkene und Alkine hydriert.

10. Verfahren zur Hydroformylierung von Alkenen, dadurch gekennzeichnet, daß man hierzu Übergangsmetallkomplexe nach den Ansprüchen 1, 3, 4, 5, 6 und 7 als Katalysatoren einsetzt oder diese in situ aus den Übergangsmetallkomplexen nach Anspruch 2 herstellt.

11. Verfahren zur Cyclotrimerisierung von Acetylenverbindungen, dadurch gekennzeichnet, daß man hierzu Übergangsmetallkomplexe nach den Ansprüchen 1, 3, 4, 5, 6 und 7 als Katalysatoren einsetzt oder diese in situ aus den Übergangsmetallkomplexen nach Anspruch 2 herstellt.

## Claims

1. A transition metal complex of the formula I
[A]^{x-} [Q]⁺_{X} I
where Q is one equivalent of a cation, x is from 0 to 2 and A is a transition metal complex of the formula 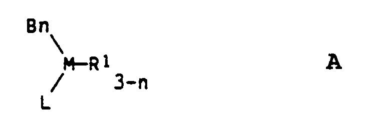 where n is from 1 to 3, M is positively charged cobalt, rhodium, iridium or ruthenium, the groups B are identical or different groups of the formula II 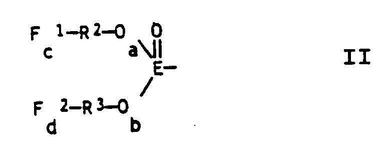 where E is phosphorus or arsenic, O is oxygen, R² and R³ are each C₁-C₂₀-alkylene which in turn may carry up to two cycloalkyl, heterocycloalkyl, aryl or hetaryl groups having a total of not more than 15 ring atoms, and R² and R³ may be interrupted by oxygen, sulfur or (-NR⁴), R⁴ is hydrogen or C₁-C₄-alkyl and the heteroatoms or heteroatom groups are each separated by two or more carbon atoms and the radicals R⁴ derived from two different (-NR⁴) groups may furthermore be bonded to one another to form a 5-membered or 6-membered ring, F¹ and F² are each alkenyl, alkynyl, C₅-C₁₂-cycloalkadienyl, nitrile, amino, C₁-C₄-mono- and dialkylamino, C₁-C₄-acyl, hydroxyl, C₁-C₄-alkoxy, aryloxy, C₁-C₄-alkylsulfido, arylsulfido, C₁-C₄-dialkyl- and diarylphosphido and/or carboxylato, and, if c or d is 0, hydrogen, a-d are each 0 or 1, with the proviso that a+b>0 and c+d>0, L is 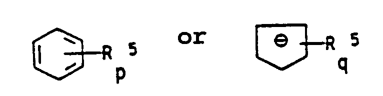 the radicals R⁵ are identical or different radicals from the group consisting of C₁-C₄-alkyl and phenyl, p is an integer from 0 to 6 and q is an integer from 0 to 5, and R¹ is fluorine, chlorine, bromine, iodine, cyanide, isocyanide, cyanate, isocyanate, thiocyanate, isothiocyanate, C₁-C₄-alkyl, C₁-C₄-trialkyl phosphite or triaryl phosphite.

2. A transition metal complex as claimed in claim 1, wherein Q is lithium, sodium, potassium, silver, copper, rhodium, iridium, ruthenium, calcium, magnesium or an ammonium or phosphonium cation.

3. A transition metal complex as claimed in claim 1, which is of the formula III
[L-Co-B₃]⁻[Q]⁺ III
where E of the group II is phosphorus.

4. A transition metal complex as claimed in claim 1, which is of the formula IV 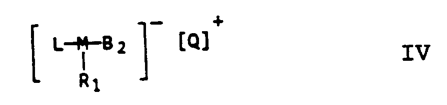 where M is selected from the group consisting of rhodium, iridium and ruthenium cations and E of the group B is phosphorus.

5. A transition metal complex as claimed in any of claims 1, 3 and 4, wherein c is 0 and R³ is C₁-C₁₆-alkylene.

6. A transition metal complex as claimed in claim 1 of the formula I, where [Q]⁺ is a complex cation of the type V
[M^{Q}Z]^{x⊕} V
where X is 1 or 2, M^{Q} is rhodium, iridium, ruthenium, chromium, tungsten, molybdenum, manganese, rhenium or copper, Z is one of the polydentate ligands C₇-cycloalkatriene, C₅-C₁₂-cycloalkadiene, C₄-C₈-alkadiene, arene or a C₂-C₆-alkylenediphosphido ligand of the formula VI
R⁶-P-(CH₂)₁₋₆-P-R⁶ VI
where R⁶ is C₁-C₄-alkyl and/or phenyl, and/or from 1 to 3 identical or different ligands selected from the group consisting of the monodentate ligands carbonyl, nitrosyl, chlorine, bromine, iodine, nitrile, isonitrile, C₁-C₄-alkyl, C₁-C₄-acyl, C₁-C₄-alkene, C₁-C₄-alkyne, C₁-C₄-trialkylphosphino, triarylphosphino and/or carbyne ligands.

7. A transition metal complex as claimed in claim 1, in which [Q]⁺ is a complex dication of the formula VII
[M^{Q}-K-M^{Q}]²⁺ VII
where M^{Q} is as defined in claim 6 and the ligands K are from 1 to 3 of the bridging ligands from the group consisting of carbonyl, C₄-C₆-alkadienyl, C₈-C₁₂-cycloalkadiene, C₁-C₄-alkylisonitrile, C₁-C₄-dialkylphosphido, diarylphosphido and/or C₁-C₆-alkylenediphosphido ligands of the formula VI.

8. A process for the catalytic hydrogenation of an unsaturated compound, wherein a transition metal complex as claimed in any of claims 1, 3, 4, 5, 6 and 7 is used as a catalyst for this purpose, or the catalyst is produced in situ from a transition metal complex as claimed in claim 2.

9. A process as claimed in claim 8, wherein an alkene or an alkyne is hydrogenated.

10. A process for the hydroformylation of an alkene, wherein a transition metal complex as claimed in any of claims 1, 3, 4, 5, 6, and 7 is used as a catalyst for this purpose, or the catalyst is produced in situ from a transition metal complex as claimed in claim 2.

11. A process for the cyclotrimerization of an acetylene compound, wherein a transition metal complex as claimed in any of claims 1, 3, 4, 5, 6 and 7 is used as a catalyst for this purpose, or the catalyst is produced in situ from a transition metal complex as claimed in claim 2.

## Revendications

1. Complexes de métal de transition de formule générale I
[A]^{x-} [Q]⁺_{X} I
dans laquelle Q est mis pour l'équivalent d'un cation, X a une valeur de 0 à 2 et A correspond à un complexe de métal de transition de formule générale 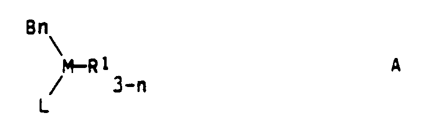 dans laquelle les variables ont la signification suivante :
n est compris entre 1 et 3
M représente du cobalt, du rhodium, de l'iridium ou du ruthénium chargés positivement
B représente des groupes identiques ou différents de formule générale II 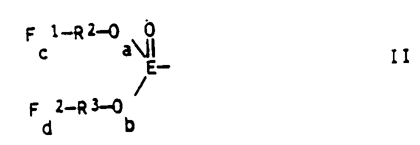 dans laquelle les variables sont définies de la manière suivante :
E représente du phosphore ou de l'arsenic,
O de l'oxygène,
R², R³ des groupes alkylène en C₁-C₂₀, qui peuvent porter pour leur part jusqu'à deux groupes cycloalkyle, hétérocycloalkyle, aryle ou hétéroaryle ayant au total jusqu'à 15 atomes sur les cycles, et les restes R² et R³ pouvant être interrompus par l'oxygène, le soufre ou des groupes -NR⁴, R⁴ signifiant un atome d'hydrogène ou un groupe alkyle en C₁-C₄ et les hétéroatomes ou les groupements comportant un hétéroatome étant chaque fois séparés par au moins deux atomes de carbone, les restes R⁴ provenant de deux groupements -NR⁴ différents pouvant également être reliés l'un à l'autre par l'intermédiaire d'un cycle à 5 ou 6 atomes,
F¹, F² sont des groupes alcényle, alcynyle, cycloalcadiényle en C₅-C₁₂, nitrile, amino, mono- et di(alkyle en C₁-C₄)amino, acyle en C₁-C₄, hydroxyle, alcoxy en C₁-C₄, aryloxy, alkylsulfido en C₁-C₄, arylsulfido, di(alkyle en C₁-C₄)- et diarylphosphido et/ou carboxylato, et dans le cas où c ou d valent 0, ils représentent un atome d'hydrogène,
a - d sont 0 ou 1, à condition que a+b>0 et c+d>0
L 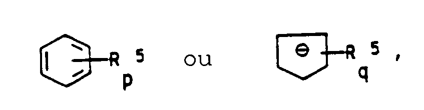 dans lesquelles R⁵ est mis pour des restes identiques ou différents parmi les groupes alkyle en C₁-C₄ ou phényle, p représente un nombre entier compris entre 0 et 6 et q un nombre entier de 0 à 5,
R¹ est un atome de fluor, de chlore, de brome, d'iode, un groupe cyanure, isocyanure, cyanate, isocyanate, thiocyanate, isothiocyanate, alkyle en C₁-C₄, tri(alkyle en C₁-C₄)- ou triarylphosphite.

2. Complexes de métal de transition selon la revendication 1, dans lesquels Q représente le lithium, le sodium, le potassium, l'argent, le cuivre, le rhodium, l'iridium, le ruthénium, le calcium, le magnésium ou un cation ammonium ou phosphonium.

3. Complexes de métal de transition selon la revendication 1, de formule générale III
[L-Co-B₃]⁻[Q]⁺ III
dans laquelle E dans le groupement II représente le phosphore.

4. Complexes de métal de transition selon la revendication 1, de formule générale IV. 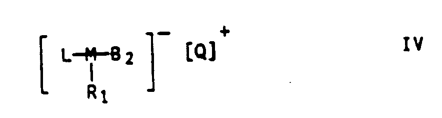 dans lesquels M est choisi parmi des cations de rhodium, d'iridium et de ruthénium et E dans le groupement B représente le phosphore.

5. Complexes de métal de transition selon l'une quelconque des revendications 1, 3 et 4, dans lesquels c est 0 et R³ est choisi parmi les groupes alkylène en C₁-C₁₆.

6. Complexes de métal de transition selon la revendication 1, de formule générale I, dans lesquels [Q]⁺_{X} est un cation complexe du type V
[M^{Q}Z]^{x⊕} V
dans lequel
X a une valeur de 1 ou 2,
M^{Q} est mis pour le rhodium, l'iridium, le ruthénium, le chrome, le tungstène, le molybdène, le manganèse, le rhénium ou le cuivre,
Z est mis pour l'un des ligands polydentés cycloalcatriéne en C₇, cycloalcadiène en C₅-C₁₂, alcadiène en C₄-C₈, arène ou pour un ligand alkylènedipbosphido en C₂-C₆ de formule générale VI
R⁶-P-(CH₂)₁₋₆-P-R⁶ VI,
dans laquelle R⁶ représente un reste alkyle en C₁-C₄ et/ou phényle, et/ou est mis pour 1 à 3 ligands monodentés identiques ou différents choisis parmi les ligands carbonyle, nitrosyle, chloro, bromo, iodo, nitrile, isonitrile, alkyle en C₁-C₄, acyle en C₁-C₄ alcène en C₁-C₄, alcyne en C₁-C₄, tri(alkyle en C₁-C₄)phosphino, triarylphosphino et/ou carbine.

7. Complexes de métal de transition selon la revendication 1, dans lesquels [Q]⁺_{X} est mis pour un dication complexe de formule générale VII
[M^{Q}-K-M^{Q}]²⁺ VII
dans laquelle M^{Q} a la signification mentionnée dans la revendication 6, K représente 1 a 3 des ligands pontants carbonyle, alcadiène en C₄-C₆, cycloalcadiène en C₈-C₁₂, alkylisonitrile en C₁-C₄, di(alkyle en C₁-C₄)phosphido, diarylphosphido et/ou des ligands alkylènediphosphido en C₁-C₆ de formule générale VI.

8. Procédé d'hydrogénation catalytique de composés insaturés, caractérisé en ce que l'on utilise à cette fin des complexes de métal de transition selon l'une quelconque des revendications 1, 3, 4, 5, 6 et 7 comme catalyseurs ou en ce que l'on prépare ceux-ci in situ à partir des complexes de métal de transition selon la revendication 2.

9. Procédé selon la revendication 8, caractérisé en ce que l'on effectue l'hydrogénation d'alcènes et d'alcynes.

10. Procédé d'hydroformylation d'alcènes, caractérisé en ce que l'on utilise a cette fin des complexes de métal de transition selon l'une quelconque des revendications 1, 3, 4, 5, 6 et 7 comme catalyseurs ou en ce que l'on prépare ceux-ci in situ à partir des complexes de métal de transition selon la revendication 2.

11. Procédé de cyclotrimérisation de composés acétyléniques, caractérisé en ce que l'on utilise a cette fin des complexes de métal de transition selon l'une quelconque des revendications 1, 3, 4, 5, 6 et 7 comme catalyseurs ou en ce que l'on prépare ceux-ci in situ à partir des complexes de métal de transition selon la revendication 2.
